(19) Europäisches Patentamt
European Patent Office
Office européen des brevets

(11) Veröffentlichungsnummer: **0 255 894 B1**

(12) **EUROPÄISCHE PATENTSCHRIFT**

(45) Veröffentlichungstag der Patentschrift: **07.10.92**

(51) Int. Cl.$^5$: **C07H 19/073**, A61K 31/70

(21) Anmeldenummer: **87110571.4**

(22) Anmeldetag: **21.07.87**

Die Akte enthält technische Angaben, die nach dem Eingang der Anmeldung eingereicht wurden und die nicht in dieser Patentschrift enthalten sind.

(54) **Pyrimidinderivate, deren Herstellung und Arzneimittel, die diese Derivate enthalten.**

(30) Priorität: **08.08.86 GB 8619424**
**07.05.87 GB 8710777**

(43) Veröffentlichungstag der Anmeldung:
**17.02.88 Patentblatt 88/07**

(45) Bekanntmachung des Hinweises auf die Patenterteilung:
**07.10.92 Patentblatt 92/41**

(84) Benannte Vertragsstaaten:
**AT BE CH DE ES FR GB GR IT LI LU NL SE**

(56) Entgegenhaltungen:
**FR-A- 2 548 190**

**CHEMICAL ABSTRACTS, Band 86, 1977, Seite 450, Zusammenfassung Nr. 16907n, Columbus, Ohio, US; & JP-A-76 86 483 (ONO PHARMACEUTICAL CO., LTD) 29-07-1976**

(73) Patentinhaber: **F. HOFFMANN-LA ROCHE AG**
**Postfach 3255**
**CH-4002 Basel(CH)**

(72) Erfinder: **Lambert, Robert Wilson,**
**33, New Road Digswell,**
**Welwyn Herts.(GB)**
Erfinder: **Martin, Joseph Armstrong,**
**10, The Chownes West Common,**
**Harpenden Herts.(GB)**
Erfinder: **Thomas, Gareth John,**
**2B, Woodland Way Oaklands,**
**Welwyn Herts.,(GB)**

(74) Vertreter: **Lederer, Franz, Dr. et al**
**Patentanwalt Dr. Franz Lederer Lucile-Grahn-Strasse 22**
**W-8000 München 80(DE)**

EP 0 255 894 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Pyrimidinderivate, ein Verfahren zu deren Herstellung und Arzneimittel, die diese Derivate enthalten.

Die erfindungsgemässen Pyrimidinderivate haben die allgemeine Formel

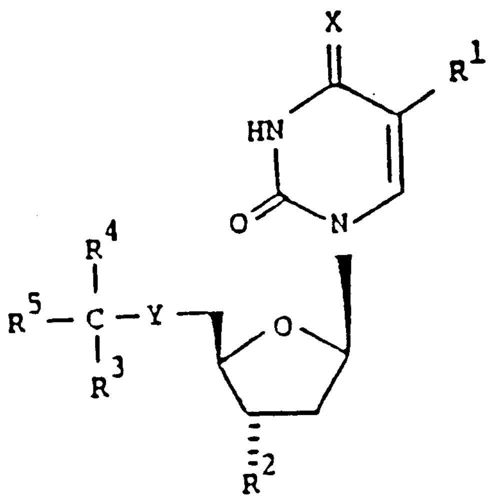

I

worin $R^1$ Halogen, $C_{1-4}$-Alkyl oder Halo-($C_{1-4}$-Alkyl), $R^2$ Wasserstoff, Hydroxy, $C_{1-4}$-Alkanoyloxy, Cyclopentylpropionyloxy, Phenacetoxy oder Bentoyloxy, $R^3$ und $R^4$ Wasserstoff oder $C_{1-4}$-Alkyl, $R^5$ Aryl oder Aryloxy, wobei Aryl gegebenenfalls durch einen oder mehrere Substituenten aus der gruppe Halogen, OH, $C_{1-4}$-Alkyl, $C_{1-4}$-Alkoxy, $CF_3$, $NO_2$ oder Phenyl substituiertes Phenyl bezeichne X Sauerstoff oder NH und Y $-CO-CH_2-$,

In der US Patentschrift 4000260 sind 5'-Amino-2',5'-dideoxyuridine und in C.A. 86: 16907n 5'-Aralkylthio-2',5'-dideoxyuridine beschrieben. Im Gegensatz zu diesen Verbindungen enthalten die vorliegenden weder eine 5'-Amino- noch eine 5'-Aralkylthiogruppe. $-CH(OH)-CH_2-$, $-CH_2-CH_2-$, -SO- oder $-SO_2-$ darstellen,

und Tautomere davon.

Der hier verwendete Ausdruck "$C_{1-4}$-Alkyl" bedeutet eine geradkettige oder verzweigtkettige Alkylgruppe wie Methyl, Aethyl, Propyl, Isopropyl, Butyl oder t-Butyl. "Halo-($C_{1-4}$-Alkyl)" bedeutet eine wie vorstehend definierte Alkylgruppe die ein oder mehrere Halogenatome trägt, z.B. Trifluormethyl oder 2-Chloräthyl. Die Acyloxygruppe kann sich von einer aliphatischen, cycloaliphatischen, araliphatischen oder aromatischen Carbonsäure ableiten; Beispiele solcher Säuren sind Ameisensäure, Essigsäure, Propionsäure, Buttersäure, Cyclopentylpropionsäure, Phenylessigsäure und Benzoesäure. Bevorzugte Acyloxygruppen sind $C_{1-4}$-Alkanoyloxygruppen. "Aryl" bedeutet unsubstituiertes Phenyl oder Phenyl, das einen oder mehrere Substituenten aus der Gruppe Halogen, Hydroxy, $C_{1-4}$-Alkyl, $C_{1-4}$-Alkoxy, Trifluormethyl, Nitro und Phenyl trägt. Beispiele solcher substituierten Phenylgruppen sind 2-Chlorphenyl, 2,4- oder 2,6-Dichlorphenyl, 2-Methylphenyl und 2,6-Dimethylphenyl. "Aryloxy" bedeutet eine wie oben definierte Arylgruppe die über ein Sauerstoffatom gebunden ist. Beispiele von Aryloxygruppen sind Phenoxy, 2-Chlorphenoxy und 2,4-Dichlorphenoxy. "Halogen" bedeutet Fluor, Chlor, Brom oder Jod.

Die Verbindungen der Formel I und deren Tautomere in denen Y $-CH(OH)-CH_2-$ darstellt oder in denen $R^3$ und $R^4$ unterschiedliche Bedeutungen haben, d.h. wenn $R^3$ Wasserstoff darstelt und $R^4$ eine $C_{1-4}$-Alkylgruppe darstellt oder wenn $R^3$ und $R^4$ verschiedene $C_{1-4}$-Alkylgruppen darstellen, enthalten ein asymmetrisches C-Atom und können infolgedessen in Form von Diastereomeren vorliegen. Die vorliegende Erfindung umfasst nicht nur die individuellen Diastereomere sondern auch Gemische davon.

In der obigen Formel I ist $R^1$ vorzugsweise $C_{1-4}$-Alkyl, insbesondere Aethyl. $R^2$ ist vorzugsweise Hydroxy oder $C_{1-4}$-Alkanoyloxy, insbesondere Hydroxy oder Acetoxy. $R^3$ und $R^4$ sind vorzugsweise Wasserstoff. $R^5$ ist vorzugsweise Dihalophenyl, insbesondere 2,6-Dichlorphenyl. X ist vorzugsweise Sauerstoff. Y ist vorzugsweise $-CO-CH_2-$, $-CH(OH)-CH_2-$, $-CH_2-CH_2-$ oder $-SO_2-$, insbesondere $-CO-CH_2-$ oder $-CH(OH)-CH_2-$.

Besonders bevorzugte erfindungsgemässe Verbindungen sind diejenigen, in denen $R^1$ Aethyl, $R^2$ Hydroxy oder Acetoxy, $R^3$ und $R^4$ Wasserstoff, $R^5$ 2,6-Dichlorphenyl, X Sauerstoff und Y $-CO-CH_2-$ oder $-CH(OH)-CH_2-$ sind.

Besonders bevorzugte Verbindungen sind:
3'-O-Acetyl-5'-[3-(2,6-dichlorphenyl)-2-oxopropyl]-2',5'-dideoxy-5-äthyluridin,
5'-[3-(2,6-Dichlorphenyl)-2-oxopropyl]-2',5'-dideoxy-5-äthyluridin und
5'[3-(2,6-Dichlorphenyl)-2(RS)-hydroxypropyl]-2',5'-dideoxy-5-äthyluridin.
Weitere bevorzugte erfindungsgemässe Verbindungen sind:
3'-O-Acetyl-2',5'-dideoxy-5-äthyl-5'-[3-(2,6-dimethylphenyl)-2-oxopropyl]uridin,
3'-O-Acetyl-2',5'-dideoxy-5-äthyl-5'-[3-(2-methylphenyl)-2-oxopropyl]uridin,
3'-O-Acetyl-5'-[3-(2-chlorophenyl)-2-oxopropyl]-2',5'-dideoxy-5-äthyluridin,
3'-O-Acetyl-2',5'-dideoxy-5-äthyl-5'-(2-oxo-3-phenylpropyl)uridin,
3'-O-Acetyl-2',5'-dideoxy-5-äthyl-5'-[2-oxo-3(RS)-phenylbutyl]uridin,
2',5'-Dideoxy-5-äthyl-5'-(2-oxo-3-phenylpropyl)uridin,
2',5'-Dideoxy-5-äthyl-5'-[3-(2-methylphenyl)-2-oxopropyl]uridin,
2',5'-Dideoxy-5-äthyl-5'-[3-(2,6-dimethylphenyl)-2-oxopropyl]uridin,
5'-[3-(2-Chlorophenyl)-2-oxopropyl]-2',5'-dideoxy-5-äthyluridin,
2',5'-Dideoxy-5-äthyl-5'-[2-oxo-3(RS)-phenylbutyl]uridin,
2',5'-Dideoxy-5-äthyl-5'-[2(RS)-hydroxy-3-phenylpropyl]uridin,
2',5'-Dideoxy-5-äthyl-5'-[2(RS)-hydroxy-3-(2-methylphenyl)propyl]uridin,
5'-[3-(2-Chlorophenyl)-2(RS)-hydroxypropyl]-2',5'-dideoxy-5-äthyluridin,
2',5'-Dideoxy-5-äthyl-5'-[2(RS)-hydroxy-3-(2,6-dimethylphenyl)propyl]uridin,
2',5'-Dideoxy-5-äthyl-5'-(3-phenylpropyl)uridin und
5'-Benzylsulphonyl-2',5'-dideoxy-5-äthyluridin.
Andere interessante Verbindungen sind:
2',5'-Dideoxy-5'-[3-(2,6-dimethylphenyl)-2-oxopropyl]uridin,
3'-O-Acetyl-5'-[3(RS)-(2,4-dichlorphenoxy)-2-oxobutyl]2',5'-dideoxy-5-äthyluridin,
5'-[3(RS)-(2,4-Dichlorphenoxy)-2-oxobutyl]-2',5'-dideoxy-5-äthyluridin,
5'-[3(RS)-(2,4-Dichlorphenoxy)-2(RS)-hydroxybutyl]-2',5'-dideoxy-5-äthyluridin,
5'-(2-Chlorobenzylsulphonyl)-2',5'-dideoxy-5-äthyluridine,
5'-(2,4-Dichlorbenzylsulphonyl)-2',5'-dideoxy-5-äthyluridin und
5'-(2,6-Dichlorbenzylsulphonyl)-2',5'-dideoxy-5-äthyluridin

Die Verbindungen der Formel I und deren Tautomere können erfindungsgemäss dadurch hergestellt werden, dass man

a) zur Herstellung einer Verbindung der Formel I oder einem Tautomer davon, worin $R^2$ Wasserstoff oder Acyloxy und Y -CO-$CH_2$- ist, eine Verbindung der Formel

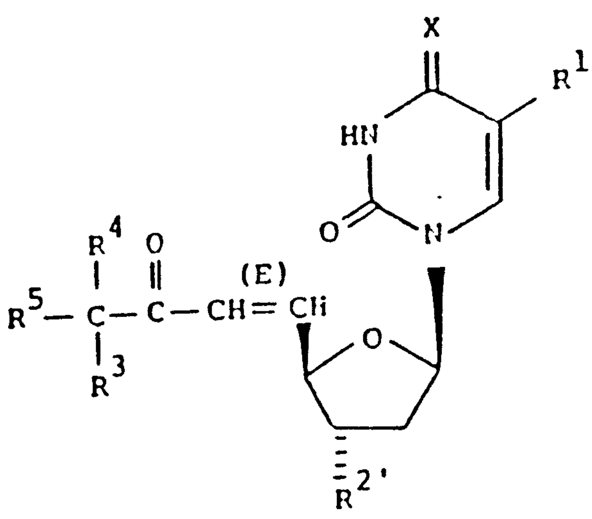

II

worin $R^1$, $R^3$, $R^4$, $R^5$ und X die obige Bedeutung haben und $R^{2'}$ Wasserstoff oder Acyloxy ist, oder ein Tautomer davon, katalytisch hydriert oder

b) zur Herstellung einer Verbindung der Formel I oder einem Tautomer davon, worin $R^2$ Wasserstoff oder Acyloxy und Y -CH(OH)-$CH_2$- ist, eine Verbindung der Formel I oder ein Tautomer davon, worin $R^2$ Wasserstoff oder Acyloxy und Y -CO-$CH_2$- ist, mit einem komplexen Metallhydrid reduziert oder

c) zur Herstellung einer Verbindung der Formel I oder einem Tautomer davon, worin $R^2$ Wasserstoff oder Acyloxy und Y -$CH_2$-$CH_2$- ist, die Hydroxygruppe in einer Verbindung der Formel I oder einem Tautomer

davon, worin $R^2$ Wasserstoff oder Acyloxy und Y $-CH(OH)-CH_2-$ ist, durch Wasserstoff ersetzt oder
d) zur Herstellung einer Verbindung der Formel I oder einem Tautomer davon, worin Y -SO- oder $-SO_2-$ ist, eine Verbindung der Formel

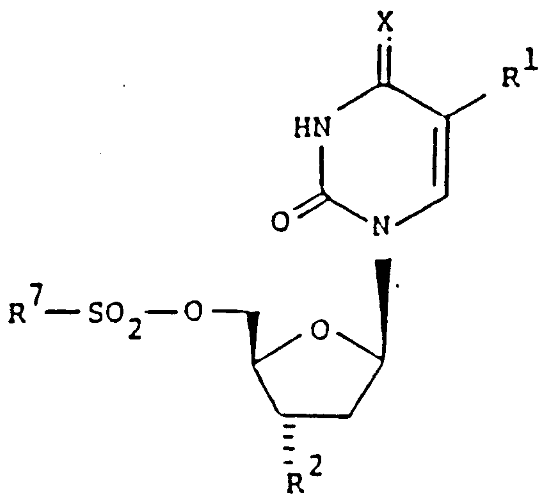

III

worin $R^1$, $R^2$ und Y die obige Bedeutung haben und
$R^7$ $C_{1-4}$-Alkyl oder Aryl ist,
oder ein Tautomer davon mit einem Alkalimetallderivat einer Verbindung der allgemeinen Formel

$HSC(R^3,R^4,R^5)$ IV

worin $R^3$, $R^4$ und $R^5$ die obige Bedeutung haben, bei erhöhter Temperatur umsetzt und die erhaltene Verbindung der Formel I oder ein erhaltenes Tautomer davon, worin Y -S- ist, oxydiert oder
e) zur Herstellung einer Verbindung der Formel I oder einem Tautomer davon, worin $R^2$ Hydroxy ist, eine Verbindung der Formel I oder ein Tautomer davon, worin $R^2$ Acyloxy ist, deacyliert.

Die katalytische Hydrierung gemäss Verfahrensvariante a) kann in an sich bekannter Weise durchgeführt werden, z.B. in einem inerten organischen Lösungsmittel wie einem Alkanol, z.B. Methanol oder Aethanol unter Verwendung eines Edelmetallkatalysators wie Palladium oder Platinkatalysatoren die auf ein inertes Trägermaterial aufgebracht sein können. Palladium auf Kohle (Pd/C) ist der bevorzugte Katalysator. Zweckmässig wird die katalytische Hydrierung bei etwa Raumtemperatur und unter Atmosphärendruck durchgeführt.

Die Reduktion gemäss Verfahrensvariante b) kann in an sich bekannter Weise durchgeführt werden, z.B. durch Behandlung mit Natriumborhydrid oder Kaliumborhydrid oder, falls $R^2$ Wasserstoff ist, auch mit Lithiumborhydrid oder einem Alkalimetallaluminiumhydrid wie Lithiumaluminiumhydrid. Diese Behandlung wird zweckmässig in einem inerten organischen Lösungsmittel und bei Raumtemperatur bis Rückflusstemperatur des Reaktionsgemisches, vorzugsweise bei etwa Raumtemperatur durchgeführt. Wenn die Reduktion mit einem Alkalimetallborhydrid ausgeführt wird, sind geeignete Lösungsmittel Alkanole, z.B. Methanol oder Aethanol, aliphatische Aether, z.B. Diäthyläther oder Dimethoxyäthan und cyclische Aether, z.B. Tetrahydrofuran und Dioxan. Geeignete Lösungsmittel zur Durchführung der Reduktion mit einem Alkalimetallaluminiumhydrid sind aliphatische und cyclische Aether wie die früher erwähnten.

Der Ersatz von Hydroxy durch Wasserstoff gemäss Verfahrensvariante c) kann ebenfalls in an sich bekannter Weise durchgeführt werden. Beispielsweise kann die Verbindung der Formel I oder ein Tautomer davon zunächst in den entsprechenden Sulfonsäureester, wie das Mesylat, durch Behandlung mit einem Sulfonsäurehalogenid wie Methansulfonylchlorid umgewandelt werden, zweckmässig in Gegenwart eines Säurebindemittels insbesondere einem tertiären Amin wie Pyridin und bei niedriger Temperatur, z.B. bei 0 °C. Der erhaltene Sulfonsäureester kann dann in das entsprechende Jodid, z.B. durch Behandlung mit einem Alkalimetalljodid, wie Natriumjodid in Aceton, bei erhöhter Temperatur, vorzugsweise bei Rückflusstemperatur des Reaktionsgemisches umgewandelt werden. Das erhaltene Jodid kann dann in die gewünschte Verbindung der Formel I oder ein Tautomer davon, worin Y $-CH_2-CH_2-$ ist, durch katalytische Hydrierung in an sich bekannter Weise, z.B. mittels Palladium auf Bariumsulfat umgewandelt werden.

Bei der Reaktion einer Verbindung der Formel III oder einem Tautomeren davon mit einem Alkalimetallderivat vorzugsweise dem Natriumderivat, einer Verbindung der Formel IV gemäss Verfahrensvariante d)

wird die $R^7$-$SO_3$-Gruppe durch die -SC($R^3$,$R^4$,$R^5$)-Gruppe verdrängt. Die Reaktion wird zweckmässig in Gegenwart eines inerten organischen Lösungsmittels wie Dimethylformamid und bei etwa 100 °C durchgeführt. Das Alkalimetallderivat wird zweckmässig in situ aus der Verbindung der Formel IV und einem Alkalimetallhydrid wie Natriumhydrid gebildet.

Die Oxidation der erhaltenen Verbindung kann ebenfalls in an sich bekannter Weise ausgeführt werden. Beispielsweise wird eine Verbindung der Formel III oder ein Tautomer davon mit einer organischen Persäure wie Peressigsäure, Perbenzoesäure, m-Chlorperbenzoesäure oder Perphthalsäure, zweckmässig in einem geeigneten Lösungsmittel wie einem halogenierten Kohlenwasserstoff, z.B. Chloroform oder einer Alkancarbonsäure, z.B. Essigsäure, und einer Temperatur zwischen etwa 0 °C und Raumtemperatur behandelt. Wenn Peressigsäure für die Oxidation verwendet wird, wird diese zweckmässig in situ als Eisessig und Wasserstoffperoxid hergestellt. Wenn ein Aequivalent einer organischen Persäure angewandt wird, erhält man eine Verbindung der Formel I oder ein Tautomer davon, worin Y -SO- darstellt, wogegen die Verwendung von 2 Aequivalenten organischer Persäure zur Verbindung der Formel I oder einem Tautomer davon führt, worin Y -$SO_2$- darstellt.

Die Deacylierung gemäss Verfahrensvariante e) kann in an sich bekannter Weise, z.B. durch Behandlung mit einem Alkalimetall $C_{1-4}$-Alkoxid wie Natriummethoxid in einem $C_{1-4}$-Alkanol wie Methanol durchgeführt werden. Zweckmässig wird diese Behandlung bei Raumtemperatur durchgeführt, obgleich sie gewünschtenfalls bei erhöhter Temperatur durchgeführt werden kann.

Die Verbindungen der Formel II und deren Tautomere die als Ausgangsmaterial in der Verfahrensvariante a) eingesetzt werden, sind neu und bilden ebenfalls Gegenstand der vorliegenden Erfindung. Sie können beispielsweise dadurch hergestellt werden, dass man zunächst eine Verbindung der Formel

$ClCH_2COC(R^3,R^4,R^5)$ V

worin $R^3$, $R^4$ und $R^5$ die obige Bedeutung haben, mit einem Triarylphosphin vorzugsweise Triphenylphosphin, zweckmässig in einem inerten organischen Lösungsmittel wie einem halogenierten Kohlenwasserstoff, z.B. Chloroform und bei erhöhter Temperatur zweckmässig bei Rückflusstemperatur des Reaktionsgemisches zu einem Phosphoniumchlorid der allgemeinen Formel

$Cl^-[(R^6)_3PCH_2COC(R^3,R^4,R^5)]^+$ VI

worin $R^3$, $R^4$ und $R^5$ die obige Bedeutung haben und
$R^6$ Aryl ist,
umsetzt.

Das Phosphoniumchlorid der Formel VI wird dann mit einer starken anorganischen Base wie einem Alkalimetallhydrid, z.B. Natriumhydrid oder einem Alkalimetallhydroxid, z.B. Natriumhydroxid behandelt und das erhaltene Phosphoran der allgemeinen Formel

$(R^6)_3P=CHCOC(R^3,R^4,R^5)$ VII

worin $R^3$, $R^4$, $R^5$ und $R^6$ die obige Bedeutung haben,
wird dann mit einer Verbindung der allgemeinen Formel

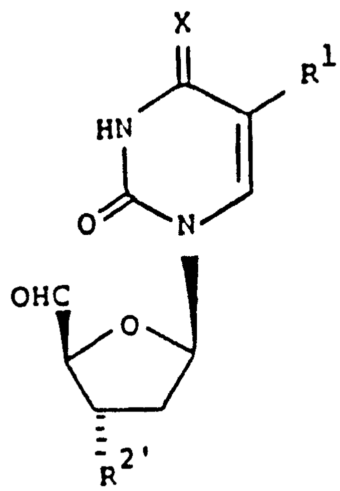

VIII

worin $R^1$, $R^{2'}$ und X die obige Bedeutung haben,
oder einem Tautomer davon unter den Bedingungen einer Wittig-Reaktion zu einer Verbindung der Formel II umgesetzt.

Die Verbindungen der Formel V, die zur Herstellung der Verbindung der Formel II und deren Tautomeren gebraucht werden, sind bekannte Verbindungen oder Analoge bekannter Verbindungen, die in ähnlicher Weise wie die bekannten Verbindungen hergestellt werden können. Die Verbindungen der Formel III und deren Tautomere, die als Ausgangsmaterial in Verfahrensvariante d) angewandt werden, sind bekannte Verbindungen oder Analoge bekannter Verbindungen die in ähnlicher Weise wie die bekannten Verbindungen hergestellt werden können.

Die Verbindung der Formel I und deren Tautomere besitzen antivirale Wirkung und können zur Kontrolle oder Verhütung viraler Infektionen, beispielsweise Herpes simplex-Virus-Infektionen verwendet werden. Die in vitro Aktivität dieser Verbindung bei der Hemmung von Herpes simplex-Virus Typ 2 (HSV-2)-Thymidinkinase kann mittels des folgenden Tests gezeigt werden:

In diesem Test enthält ein Versuchsgemisch 50 mM Tris-HCl, pH 8, 5 mM Magnesiumchlorid, 5 mM ATP, 0,3 $\mu$M $^3$H-Thymidin (50 Ci/mMol), in geeigneter Weise verdünnten Thymidinkinaseextrakt und verschiedene Konzentrationen von Verbindungen der Formel I oder Tautomeren davon in einem Gesamtvolumen von 100 $\mu$l. Die Testlösungen werden 30 Minuten bei 37°C inkubiert und die Reaktion durch 2 Minuten langes Eintauchen in kochendes Wasser beendet. Von jeder Testlösung werden dann 85 $\mu$l auf Cellulosepapier-Discs getrocknet und das unphosphorylierte $^3$H-Thymidin durch Waschen in 4 mM Ammoniumformiat entfernt. Die an die Discs gebunden gebliebene Radioaktivität wird dann durch Szintillationsspektrophotometrie gemessen. Der Grad der Hemmung bei jeder Konzentration der Verbindung der Formel I wird als Prozentsatz der Kontrollreaktion (100%) nach Abzug eines gemessenen Blindwertes ausgedrückt, der die Menge Radioaktivität darstellt, die an die Discs aus einem Versuch mit Hitze-inaktivierten Enzymen gebunden wird. Der $IC_{50}$-Wert, nämlich die Konzentration der Verbindung der Formel I oder dessen Tautomeren, die die Enzymaktivität zu 50% hemmt, wird dann berechnet. Die mit repräsentativen Verbindungen der Formel I erhaltenen Resultate sind in der nachfolgenden Tabelle zusammengestellt:

Tabelle

| Verbindung von Beispiel No. | $IC_{50}$ ($\mu$M) |
|---|---|
| 3 | 0.0024 |
| 5 | 0.016 |
| 7 | 0.17 |
| 8 b | 0.6 |

Die Verbindungen der Formel I und deren Tautomere können in Form pharmazeutischer Präparate, die diese Verbindungen zusammen mit einem verträglichen pharmazeutischen Träger enthalten, als Medikamente verwendet werden. Die Träger können organische oder anorganische Materialien sein, die für enterale, z.B. orale, oder parenterale Verabreichung geeignet sind. Beispiele solcher Träger sind Wasser,

Gelatine, Gummi arabicum, Lactose, Stärke, Magnesiumstearat, Talkum, vegetabile Oele, Polyalkylenglykole und Vaseline. Die pharmazeutischen Präparate können in fester Form, z.B. als Tabletten, Dragées, Suppositorien oder Kapseln, oder in flüssiger Form, z.B. als Lösungen, Suspensionen oder Emulsionen vorliegen; sie können üblichen pharmazeutischen Operationen, z.B. Sterilisierung unterworfen werden und/oder können Hilfsstoffe, z.B. Konservierungs-, Stabilisierungs-, Befeuchtungsmittel oder Emulgatoren, Salze zur Veränderung des osmotischen Druckes oder Puffer enthalten. Sie können auch andere therapeutisch wertvolle Substanzen enthalten.

Die Verbindungen der Formel I und deren Tautomere können an Erwachsene in einer täglichen Dosierung von etwa 1-1000 mg, vorzugsweise etwa 5-500 mg verabreicht werden. Die tägliche Dosis kann in einer Einzeldosis oder in Teildosen verabreicht werden. Der obige Dosierungsbereich ist nur beispielhaft zu verstehen und kann nach oben und unten in Abhängigkeit von Faktoren wie der einzelnen zu verabreichenden Verbindung, der Verabreichungsart, der Schwere der zu behandelnden Erkrankung und dem Zustand des Patienten variiert werden.

Beispiel 1

Eine Lösung von 3,30 g (E)-3'-O-Acetyl-5'-[3-(2,6-dichlorphenyl) -2-oxopropyliden]-2',5'-dideoxy -5-äthyluridin in 1,50 l Methanol wurde über 1,10 g 10% Pd/C Katalysator bei Raumtemperatur und unter Atmosphärendruck 3 Stunden hydriert. Das Gemisch wurde abfiltriert und das Filtrat eingedampft. Der Rückstand wurde mit Diäthyläther verrieben und lieferte 2,45 g 3'-O-Acetyl-5'-[3-(2,6--dichlorphenyl) -2-oxopropyl]-2',5'-dideoxy-5-äthyluridin, Schmelzpunkt 186 ° C.

Das Ausgangsmaterial wurde wie folgt hergestellt:

(A) 50 ml Oxalylchlorid und 0,5 ml Dimethylformamid wurden zu einer gerührten Suspension von 5,12 g (2,6-Dichlorphenyl)essigsäure in 120 ml Toluol gegeben. Das Gemisch wurde 2,5 Stunden bei Raumtemperatur gerührt und dann zur Trockene eingedampft. Der Rückstand wurde in 40 ml Diäthyläther suspendiert und die Suspension nach und nach zu 250 ml einer 0,25M Lösung von Diazomethan in Diäthyläther gegeben. Das Gemisch wurde 2 Stunden bei Raumtemperatur gerührt und dann auf 0 ° C gekühlt. Dann wurde 10 Minuten lang Chlorwasserstoff durch das Gemisch geblasen. Das Gemisch wurde dann mit 300 ml Wasser versetzt und die Phasen getrennt. Die organische Phase wurde mit 200 ml gesättigter Natriumhydrogencarbonatlösung und 300 ml Wasser gewaschen, über Natriumsulfat getrocknet und eingedampft. Man erhielt 6,04 g 1-Chlor-3-(2,6-dichlorphenyl) -2-propanon in Form eines weissen Feststoffs. Dieser Feststoff wurde in 21 ml Chloroform aufgenommen, mit 7,19 g Triphenylphosphin versetzt und 6 Stunden unter Rühren zum Rückfluss erhitzt. Das Gemisch wurde gekühlt und in 200 ml Diäthyläther gegossen. Der erhaltene Niederschlag wurde gesammelt, mit Diäthyläther gewaschen und getrocknet und lieferte 8,605 g [3-(2,6-Dichlorphenyl) -2-oxo-propyl]triphenylphosphoniumchlorid als weissen Feststoff. Dieser Feststoff wurde in 1,5 l warmem Wasser aufgenommen und das Gemisch filtriert. Das Filtrat wurde unter Rühren mit 12,5 ml 5% Natriumhydroxidlösung versetzt. Das Gemisch wurde 2 mal mit je 600 ml Diäthyläther extrahiert und die vereinigten Extrakte mit einem Liter Wasser gewaschen, über Natriumsulfat getrocknet und eingedampft. Der Rückstand wurde aus 150 ml Diäthyläther kristallisiert und lieferte 4,286 g [3-(2,6-Dichlorphenyl) -2-oxo-propyliden]triphenylphosphoran vom Schmelzpunkt 98-100 ° C.

(B) Eine Lösung von 2,759 g 3'-O-Acetyl-2'-deoxy-5-äthyluridin, 5,75 g Dicyclohexylcarbodiimid und 0,375 ml Dichloressigsäure in 24 ml Dimethylsulfoxid wurde 27 Stunden bei Raumtemperatur gerührt. Danach wurden 0,375 ml Pyridin und 4,286 g [3-(2,6-Dichlorphenyl) -2-oxo-propyliden]-triphenylphosphoran zugesetzt und das Gemisch weitere 23 Stunden gerührt. Das Gemisch wurde filtriert und das Filtrat eingedampft. Der Rückstand wurde in 100 ml Aethylacetat gelöst und die Lösung 2 mal mit je 100 ml Wasser gewaschen, über Natriumsulfat getrocknet und eingedampft. Der erhaltene Gummi wurde durch Flash-Chromatographie an einer Silicagelsäule mit Aethylacetat/Hexan (2:1) gereinigt. Man erhielt 3,84 g (E)-3'-O-Acetyl-5'-[3-(2,6-dichlorphenyl) -2-oxopropyliden]-2',5'-dideoxy-5-äthyluridin als weissen Feststoff vom Schmelzpunkt 165 ° C.

Beispiel 2

In Analogie zu Beispiel 1 wurden erhalten:

a) aus (E)-3'-O-Acetyl-2',5'-dideoxy-5-äthyl -5'-[3-(2,6-dimethylphenyl) -2-oxopropyliden]uridin, Schmelzpunkt 126-130 ° C (hergestellt in Analogie zu Beispiel 1A) und B) ausgehend von (2,6-Dimethylphenyl)-essigsäure):

3'-O-Acetyl-2',5'-dideoxy-5 -äthyl-5'-[3-(2,6-dimethylphenyl)-2-oxopropyl]uridin, Schmelzpunkt 150-150,5 ° C

b) aus (E)-3'-O-Acetyl-2',5'-dideoxy-5-äthyl-5'-[3-(2-methylphenyl)-2-oxopropyliden]uridin, Schmelzpunkt 124-126 ° C, (hergestellt aus (2-Methylphenyl)essigsäure):

3'-O-Acetyl-2',5'-dideoxy-5-äthyl-5' -[3-(2-methylphenyl)-2-oxopropyl]uridin, Schmelzpunkt 156,5 ° C

c) aus (E)-3'-O-Acetyl-5' -[3-(2-chlorphenyl)-2-oxopropyliden]-2',5'-dideoxy-5-äthyluridin, Schmelzpunkt 144-145 ° C, (hergestellt aus (2-Chlorophenyl)essigsäure):

3'-O-Acetyl-5'-[3-(2-chlorphenyl)-2-oxopropyl]-2',5' -dideoxy-5-äthyluridin, Schmelzpunkt 168,5 ° C

d) aus (E)-3'-O-Acetyl-2',5'-dideoxy-5-äthyl-5' -(2-oxo-3-phenylpropyliden)uridin, (hergestellt aus Phenylessigsäure):

3'-O-Acetyl-2',5'-dideoxy-5 -äthyl-5'-(2-oxo-3-phenylpropyl)uridin, NMR ($CDCl_3$); δ1.10 (t,3), 1,85-2,03 (m,2), 2,10 (s,3), 2,10-2,18 (m,1), 2,30-2,40 (m,3), 2,57-2,75 (m,2), 3,72 (s,2), 3,93 (m,1), 4,97 (m,1), 6,27 (dd,1), 7,03 (s,1), 7,17-7,35 (m,5), 8,83 (s,1)

e) aus (E)-3'-O-Acetyl-2',5'-dideoxy-5-äthyl-5' -(2-oxo-3-phenylbutyliden)uridin, (hergestellt aus 2-Phenylpropansäure:

3'-O-Acetyl-2',5'-dideoxy-5-äthyl-5'-[2-oxo-3(RS) -phenylbutyl]uridin, NMR ($CDCl_3$); δ1,07-1,17 (dt,3), 1,35-1,42 (dd,3), 1,72-2,02 (m,2), 2,05-2,13 (m,1), 2,07(s,3), 2,30-2,43 (m,3), 2,50-2,60 (m,2), 3,72-3,82 (m,1), 3,89 (m,1), 4,94 (m,1), 6,20 (m,1), 7,01 (d,1), 7,19-7,35 (m,5), 8,52 (d,1).

Beispiel 3

Eine Lösung von 2 g 3'-O-Acetyl-5'-[3-(2,6-dichlorphenyl) -2-oxopropyl]-2',5'-dideoxy-5'-äthyluridin in 45 ml 0,1M methanolischer Natriummethoxidlösung wurde 1,5 Stunden bei Raumtemperatur gerührt. Die Lösung wurde mit 500 ml Methanol verdünnt, ein Polystyrol-Divinylbenzolkationenaustauscherharz mit Sulfonsäuregruppen ($H^+$-Form) zugesetzt, das Gemisch 10 Minuten gerührt und filtriert. Das Filtrat wurde eingedampft und der Rückstand mit Diäthyläther verrieben, wobei 1,72 g 5'-[3-(2,6-Dichlorphenyl) -2-oxopropyl)-2',5'-dideoxy -5-äthyluridin, Schmelzpunkt 229-230 ° C erhalten wurden.

Beispiel 4

In Analogie zu Beispiel 3 wurden erhalten:

a) aus 3'-O-Acetyl-2',5'-dideoxy-5 -äthyl-5'-(2-oxo-3-phenylpropyl)uridin:

2',5'-Dideoxy-5-äthyl-5'-(2-oxo-3-phenylpropyl)uridin, Schmelzpunkt 148-151 ° C

b) aus 3'-O-Acetyl-2',5'-dideoxy-5 -äthyl-5'-[3-(2-methylphenyl)-2-oxopropyl]uridin:

2',5'-Dideoxy-5-äthyl-5' -[3-(2-methylphenyl)-2-oxopropyl]uridin, Schmelzpunkt 165 ° C

c) aus 3'-O-Acetyl-2',5'-dideoxy-5 -äthyl-5'-[3-(2,6-dimethylphenyl)-2-oxopropyl]uridin:

2',5'-Dideoxy-5-äthyl-5' -[3-(2,6-dimethylphenyl)-2-oxopropyl]uridin, Schmelzpunkt 223-224 ° C

d) aus 3'-O-Acetyl-5'-[3-(2-chlorophenyl)-2 -oxopropyl]-2',5'-dideoxy-5-äthyluridin:

5'-[3-(2-Chlorophenyl)-2 -oxopropyl]-2',5' -dideoxy-5-äthyluridin, Schmelzpunkt 180-181 ° C

e) aus 3'-O-Acetyl-2',5'-dideoxy-5 -äthyl-5'-[2-oxo-3(RS)-phenylbutyl]uridin:

2',5'-Dideoxy-5-äthyl-5' -[2-oxo-3(RS)-phenylbutyl]uridin, Schmelzpunkt 145 ° C.

Beispiel 5

Eine Lösung von 149 mg 3'-O-Acetyl-5'-[3-(2,6-dichlorphenyl) -2-oxopropyl]-2',5'-dideoxy -5-äthyluridin und 26 mg Natriumborhydrid in 7 ml Dimethoxyäthan wurde 2,5 Stunden bei Raumtemperatur gerührt. Das Lösungsmittel wurde abgedampft und der Rückstand in 22 ml 5% Ammoniumchloridlösung aufgenommen und 2 mal mit je 20 ml Aethylacetat extrahiert. Die Extrakte wurden mit 20 ml Wasser gewaschen, über Natriumsulfat getrocknet und eingedampft, wobei 3'-O-Acetyl-5'-[3-(2,6-dichlorphenyl) -2(RS)-hydroxypropyl]-2',5'-dideoxy -5-äthyluridin als farbloser Gummi erhalten wurde. Dieser wurde in 3 ml 0.1M Natriummethoxidlösung gelöst und 1 Stunde bei Raumtemperatur gerührt. Die Lösung wurde dann mit 150 ml Methanol verdünnt, mit einem quervernetzten Polystyrol/Divinylbenzolkationenaustauscherharz, das Sulfonsäuregruppen ($H^+$-Form) enthielt, gerührt und dann filtriert. Das Filtrat wurde eingedampft und der Rückstand aus Aethanol kristallisiert. Man erhielt 45 mg 5'-[3-(2,6-Dichlorphenyl) -2(RS)-hydroxypropyl]-2',5'-dideoxy -5-äthyluridin. Schmelzpunkt 185-186 ° C.

Beispiel 6

In Analogie zu Beispiel 5 wurden erhalten:

a) aus 3'-O-Acetyl-2',5'-dideoxy-5 -äthyl-5'-(3-phenyl-2-oxopropyl)uridin:
2',5'-Dideoxy-5-äthyl-5' -[2(RS)-hydroxy-3-phenylpropyl]uridin, Schmelzpunkt 142° C
b) aus 3'-O-Acetyl-2',5'-dideoxy-5 -äthyl-5'-[3-(2-methylphenyl)-2-oxopropyl]uridin:
2',5'-Dideoxy-5-äthyl-5' -[2(RS)-hydroxy-3-(2-methylphenyl)propyl]uridin, Schmelzpunkt 161,5-163° C
c) aus 3'-O-Acetyl-5'-[3-(2-chlorophenyl)-2 -oxopropyl]-2',5'-dideoxy-5-äthyluridin:
5'-[3-(2-Chlorophenyl)-2(RS) -hydroxypropyl]-2',5'-dideoxy-5-äthyluridin, Schmelzpunkt 215-217° C
d) aus 3'-O-Acetyl-2',5'-dideoxy-5 -äthyl-5'-[3-(2,6-dimethylphenyl)-2-oxopropyl]uridin:
2',5'-Dideoxy-5-äthyl-5' -[2(RS)-hydroxy-3-(2,6-dimethylphenyl)propyl]uridin, Schmelzpunkt 174-178° C.

Beispiel 7

Eine Lösung von 300 mg 3'-O-Acetyl-5'-[2(RS)-hydroxy -3-phenylpropyl]-2',5'-dideoxy -5-äthyluridin und 0,2 ml Methansulfonylchlorid in 5 ml Pyridin wurde über Nacht bei 0° C stehen gelassen. Das Gemisch wurde auf 40 ml Eiswasser gegossen, gerührt und mit 40 ml Aethylacetat extrahiert. Der Extrakt wurde über Natriumsulfat getrocknet und eingedampft und lieferte 330 mg 3'-O-Acetyl-2',5'-dideoxy -5-äthyl-5'-[2(RS)-methansulfonyloxy-3-phenylpropyl]uridin.

Ein Gemisch von 240 mg der letzteren Verbindung und 190 mg Natriumjodid in 5 ml Aceton wurde 5,5 Stunden unter Rühren zum Rückfluss erhitzt. Das Gemisch wurde abkühlen gelassen und dann filtriert. Das Filtrat wurde eingedampft und der Rückstand in 50 ml Dichlormethan aufgenommen, mit 50 ml Wasser 2 mal mit je 50 ml 5% Natriumthiosulfatlösung und 50 ml gewaschen, über Natriumsulfat getrocknet und eingedampft. Man erhielt 250 mg 3'-O-Acetyl-2',5'-dideoxy-5-äthyl -5'-[2(RS)-jod-3-phenylpropyl]uridin.

Eine Lösung von 80 mg der letztgenannten Verbindung in 5 ml Aethanol wurde mit Ammoniak gesättigt. Danach wurden 50 mg Palladium auf Bariumsulfat zugesetzt und das Gemisch 3 Tage bei Raumtemperatur unter Atmosphärendruck hydriert. Das Gemisch wurde filtriert und das Filtrat eingedampft. Der Rückstand wurde mit mehreren Portionen Aethylacetat extrahiert und die vereinigten Extrakte eingedampft. Man erhielt 70 mg 3'-O-Acetyl-2',5'-dideoxy -5-äthyl-5'-(3-phenylpropyl)uridin.

Eine Lösung von 70 mg der letztgenannten Verbindung in 2 ml 0,1M Natriummethoxidlösung wurde 1 Stunde bei Raumtemperatur gerührt. Das Gemisch wurde mit einem Polystyrol/Divinylbenzol-Kationenaustauscherharz mit freien Sulfonsäuregruppen versetzt, gerührt und filtriert. Das Filtrat wurde eingedampft und der Rückstand mit Diäthyläther verrieben, wobei 22 mg 2',5'-Dideoxy-5-äthyl-5'-(3-phenylpropyl)uridin vom Schmelzpunkt 160-162° C erhalten wurden.

Beispiel 8

a) Eine Lösung von 0,5 g Benzylmercaptan in 10 ml trockenem Dimethylformamid wurde mit 60 mg einer 80%-igen Natriumhydrid-Dispersion in Mineralöl versetzt. Nach dem die Gasentwicklung aufgehört hatte, wurden 1,64 g 2'-Deoxy-5-äthyl-5'-O-(p-toluolsulfonyl)uridin in 20 ml trockenem Dimethylformamid zugesetzt. Das Gemisch wurde unter Stickstoffatmosphäre auf 100° C unter Rührn erhitzt. Der Verlauf der Reaktion wurde durch Dünnschichtchromatographie verfolgt. Nach 4 Stunden wurde das Gemisch zu einem öligen Gemisch eingedampft. Dieser wurde durch Flash-Säulenchromatographie an Silicagel mit Methanol/Dichloräthan (1:9) gereinigt. Die Fraktionen die das Produkt enthielten wurden vereinigt und zu einem Oel eingedampft, das sich verfestigte und nach Umkristallisation aus Diäthyläther 1,46 g 5'-Benzylthio-2',5'-dideoxy -5-äthyluridin, Schmelzpunkt 149-151° C lieferte.
b) 1,5 g 5'-Benzylthio-2',5'-dideoxy -5-äthyluridin wurden in 10 ml Eisessig gelöst und die Lösung auf 0° C gekühlt, worauf 0,9 ml 30%-iges Wasserstoffperoxid zugesetzt wurde. Das Gemisch wurde 1 Stunde bei 0° C und 17 Stunden bei Raumtemperatur gerührt. Die Essigsäure wurde abgedampft und der erhaltene Feststoff aus etwa 100 ml Methanol kristallisiert, wobei 0,43 g 5'-Benzylsulfonyl-2',5'-dideoxy -5-äthyluridin vom Schmelzpunkt 232-233° C erhalten wurden.

Beispiel 9

In analoger Weise wie in Beispiel 1 wurde aus (E)-3'-O-Acetyl-5'-[3(RS)-(2,4-dichlorphenoxy) -2-oxo-butyliden]-2',5'-dideoxy-5-äthyluridin (hergestellt in Analogie zu Beispiel 1(A) und (B) aus 2(RS)-(2,4-Dichlorphenoxy)propionsäure (erhalten:

3'-O-Acetyl-5'-[3(RS)-(2,4-dichlorphenoxy)-2-oxo-butyl]-2',5'-dideoxy-5-äthyluridin vom Schmelzpunkt 122-124° C.

Beispiel 10

In Analogie zu Beispiel 3 wurde aus 3'-O-Acetyl-5'-[3(RS)-(2,4-dichlorphenoxy) -2-oxo-butyl]-2',5'-dideoxy-5-äthyluridin das 5'-(3(RS)-(2,4-Dichlorphenoxy) -2-oxo-butyl]-2',5'-dideoxy-5-äthyluridin, Schmelzpunkt 157-159 ° C erhalten.

Beispiel 11

In Analogie zu Beispiel 5 wurde aus 3'-O-Acetyl-5'-[3(RS)-(2,4-dichlorphenoxy) -2-oxo-butyl]-2',5'-dideoxy -5-äthyluridin das 5'-[3(RS)-(2,4-Dichlorphenoxy)-2-(RS)-hydroxybutyl] -2',5'-dideoxy-5-äthyluridin vom Schmelzpunkt 108-111 ° C erhalten.

Beispiel 12

In Analogie zu Beispiel 7 wurde aus 3'-O-Acetyl-2',5'--dideoxy -5-äthyl-5'-[2(RS)-hydroxy -3-(2,6-dimethylphenyl)propyl]uridin das 2',5'-Dideoxy-5-äthyl-5'-[3-(2,6-dimethylphenyl)propyl]uridin vom Schmelzpunkt 213,5-214 ° C erhalten.

Beispiel 13

A) In Analogie zu Beispiel 8a) wurden erhalten:
a) aus 2'-Deoxy-5-äthyl-5'-O-(p-toluolsulfonyl)uridin und 2-Chlorbenzylmercaptan:
5'-(2-Chlorobenzylthio)-2',5'-dideoxy-5-äthyluridin. Schmelzpunkt 147-148 ° C
b) aus 2'-Deoxy-5-äthyl-5'-O-(p-toluolsulfonyl)uridin und 2,4-Dichlorbenzylmercaptan:
5'-(2,4-Dichlorbenzylthio)-2',5'-dideoxy-5-äthyluridin, Schmelzpunkt 169-170 ° C
c) aus 2'-Deoxy-5-äthyl-5'-O-(p-toluolsulfonyl)uridin und 2,6-Dichlorbenzylmercaptan:
5'-(2,6-Dichlorbenzylthio)-2',5'-dideoxy-5-äthyluridin, Schmelzpunkt 206-207 ° C.
B) In Analogie zu Beispiel 8b) wurden erhalten:
a) aus 5'-(2-Chlorbenzylthio)-2',5'-dideoxy-5-äthyluridin:
5'-(2-Chlorbenzylsulfonyl)-2',5'-dideoxy-5-äthyluridin, Schmelzpunkt 189-190 ° C
b) aus 5'-(2,4-Dichlorbenzylthio)-2',5'-dideoxy-5-äthyluridin:
5'-(2,4-Dichlorbenzylsulfonyl)-2',5'-dideoxy-5-äthyluridin, Schmelzpunkt 217-218 ° C
c) aus 5'-(2,6-Dichlorbenzylthio)-2',5'-dideoxy-5-äthyluridin:
5'-(2,6-Dichlorbenzylsulfonyl)-2',5'-dideoxy-5-äthyluridin, Schmelzpunkt 236-237 ° C.

Da folgende Beispiel illustriert eine pharmazeutische Zubereitung die eine Verbindung der Formel I enthält:

Tabletten können die folgenden Inhaltsstoffe aufweisen:

| Inhaltsstoff | Pro Tablette |
|---|---|
| Verbindung der Formel I | 100 mg |
| Lactose | 70 mg |
| Maisstärke | 70 mg |
| Polyvinylpyrrolidon | 5 mg |
| Magnesiumstearat | 5 mg |
| Tablettengewicht | 250 mg |

**Patentansprüche**

**Patentansprüche für folgende Vertragsstaaten : BE, CH, DE, FR, GB, IT, LI, LU, NL, SE**

**1.** Verbindungen der allgemeinen Formel

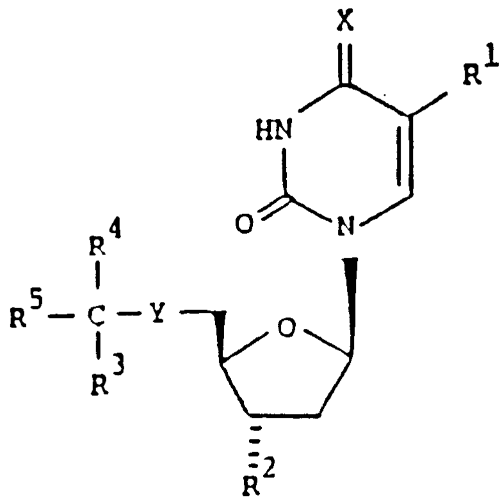

I

worin $R^1$ Halogen, $C_{1-4}$-Alkyl oder Halo-($C_{1-4}$-Alkyl), $R^2$ Wasserstoff, Hydroxy, $C_{1-4}$-Alkanoyloxy, Cyclopentylpropionyloxy, Phenacetoxy oder Benzoyloxy, $R^3$ und $R^4$ Wasserstoff oder $C_{1-4}$-Alkyl, $R^5$ Aryl oder Aryloxy, wobei Aryl gegebenenfalls durch einen oder mehrere Sustituenten aus de Gruppe Halogen, OH, $C_{1-4}$-Alkyl, $C_{1-4}$-Alkoxy, $CF_3$, $NO_2$ ode Phenyl substituiertes Phenyl bezeichnet, X Sauerstoff oder NH und Y -CO-$CH_2$-, -CH(OH)-$CH_2$-, -$CH_2CH_2$-, -SO- oder -$SO_2$-darstellen, und Tautomere davon.

**2.** Verbindungen gemäss Anspruch 1, worin $R^5$ Aryl ist.

**3.** Verbindungen gemäss Anspruch 1 oder 2, worin $R^1$ $C_{1-4}$-Alkyl ist.

**4.** Verbindungen gemäss den Ansprüchen 1-3, worin $R^2$ Hydroxy oder $C_{1-4}$-Alkanoyloxy ist.

**5.** Verbindungen gemäss den Ansprüchen 1-4, worin $R^3$ und $R^4$ Wasserstoff sind.

**6.** Verbindungen gemäss den Ansprüchen 1-5, worin $R^5$ Dihalophenyl ist.

**7.** Verbindungen gemäss den Ansprüchen 1-6, worin X Sauerstoff ist.

**8.** Verbindungen gemäss den Ansprüchen 1-7 worin Y -$COCH_2$-, -CH(OH)-$CH_2$-, -$CH_2$-$CH_2$- oder -$SO_2$- ist.

**9.** Verbindungen gemäss den Ansprüchen 1-8, worin $R^1$ Aethyl, $R^2$ Hydroxy oder Acetoxy, $R^3$ und $R^4$ Wasserstoff, $R^5$ 2,6-Dichlorphenyl, X Sauerstoff und Y -$COCH_2$- oder -CH(OH)-$CH_2$- ist.

**10.** Die Verbindungen gemäss Anspruch 1,

3'-O-Acetyl-5'-[3-(2,6-dichlorphenyl)-2-oxopropyl]-2',5'-dideoxy-5-äthyluridin,
5'-[3-(2,6-Dichlorphenyl)-2-oxopropyl]-2',5'-dideoxy-5-äthyluridin und
5'-[3-(2,6-Dichlorphenyl)-2(RS)-hydroxypropyl]-2',5'-dideoxy-5-äthyluridin.

**11.** Die Verbindungen gemäss Anspruch 2,

3'-O-Acetyl-2',5'-dideoxy-5-äthyl-5'-[3-(2,6-dimethylphenyl)-2-oxopropyl]uridin,
3'-O-Acetyl-2',5'-dideoxy-5-äthyl-5'-[3-(2-methylphenyl)-2-oxopropyl]uridin,
3'-O-Acetyl-5'-[3-(2-chlorophenyl)-2-oxopropyl]-2',5'-dideoxy-5-äthyluridin,
3'-O-Acetyl-2',5'-dideoxy-5-äthyl-5'-(2-oxo-3-phenylpropyl)uridin,
3'-O-Acetyl-2',5'-dideoxy-5-äthyl-5'-[2-oxo-3(RS)-phenylbutyl]uridin,
2',5'-Dideoxy-5-äthyl-5'-(2-oxo-3-phenylpropyl)uridin,
2',5'-Dideoxy-5-äthyl-5'-[3-(2-methylphenyl)-2-oxopropyl]uridin,
2',5'-Dideoxy-5-äthyl-5'-[3-(2,6-dimethylphenyl)-2-oxopropyl]uridin,

5'-[3-(2-Chlorophenyl)-2-oxopropyl]-2',5'-dideoxy-5-äthyluridin,
2',5'-Dideoxy-5-äthyl-5'-[2-oxo-3(RS)-phenylbutyl]uridin,
2',5'-Dideoxy-5-äthyl-5'-[2(RS)-hydroxy-3-phenylpropyl]uridin,
2',5'-Dideoxy-5-äthyl-5'-[2(RS)-hydroxy-3-(2-methylphenyl)propyl]uridin,
5'-[3-(2-Chlorophenyl)-2(RS)-hydroxypropyl]-2',5'-dideoxy-5-äthyluridin,
2',5'-Dideoxy-5-äthyl-5'-[2(RS)-hydroxy-3-(2,6-dimethylphenyl)propyl]uridin,
2',5'-Dideoxy-5-äthyl-5'-(3-phenylpropyl)uridin und
5'-Benzylsulphonyl-2',5'-dideoxy-5-äthyluridin.

**12.** Die Verbindungen gemäss Anspruch 2,

2',5'-Dideoxy-5'-[3-(2,6-dimethylphenyl)-2-oxopropyl]uridin,
3'-O-Acetyl-5'-[3(RS)-(2,4-dichlorphenoxy)-2-oxobutyl]2',5'-dideoxy-5-äthyluridin,
5'-[3(RS)-(2,4-Dichlorphenoxy)-2-oxobutyl]-2',5'-dideoxy-5-äthyluridin,
5'-[3(RS)-(2,4-Dichlorphenoxy)-2(RS)-hydroxybutyl]-2',5'-dideoxy-5-äthyluridin,
5'-(2-Chlorobenzylsulphonyl)-2',5'-dideoxy-5-äthyluridin,
5'-(2,4-Dichlorbenzylsulphonyl)-2',5'-dideoxy-5-äthyluridin und
5'-(2,6-Dichlorbenzylsulphonyl)-2',5'-dideoxy-5-äthyluridin

**13.** Verbindungen der allgemeinen Formel

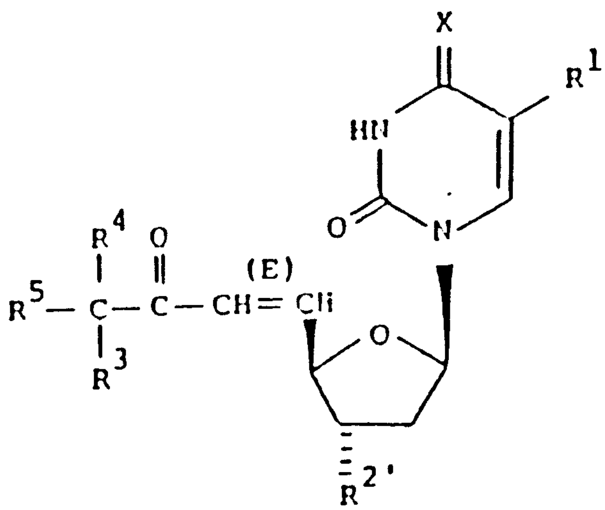

II

worin $R^1$ Halogen, $C_{1-4}$-Alkyl oder Halo-($C_{1-4}$-alkyl); $R^{2'}$ Wasserstoff oder Acyloxy; $R^3$ und $R^4$ Wasserstoff oder $C_{1-4}$-Alkyl; $R^5$ Aryl oder Aryloxy; und X Sauerstoff oder NH sind, und Tautomere davon.

**14.** Die Verbindungen gemäss den Ansprüchen 1-12 zur Verwendung als Heilmittel, insbesondere als Mittel gegen Viren.

**15.** Verfahren zur Herstellung von Verbindungen der Formel I und deren Tautomeren, dadurch gekennzeichnet, dass man

a) zur Herstellung einer Verbindung der Formel I oder einem Tautomer davon, worin $R^2$ Wasserstoff oder Acyloxy und Y -CO-$CH_2$- ist, eine Verbindung der Formel

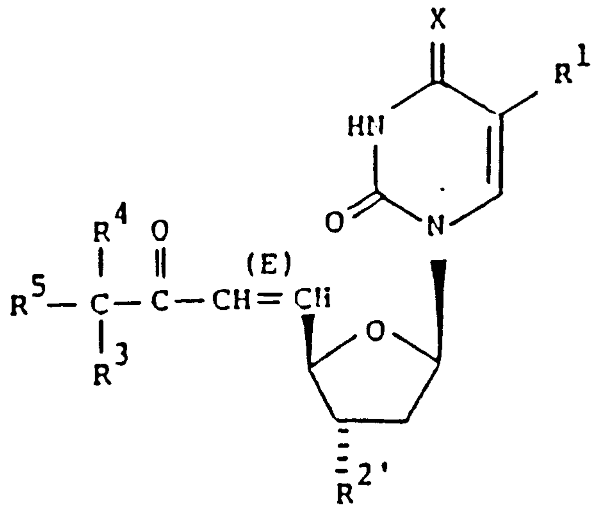

II

worin $R^1$, $R^3$, $R^4$, $R^5$ und X die obige Bedeutung haben und $R^{2'}$ Wasserstoff oder Acyloxy ist, oder ein Tautomer davon, katalytisch hydriert oder

b) zur Herstellung einer Verbindung der Formel I oder einem Tautomer davon, worin $R^2$ Wasserstoff oder Acyloxy und Y $-CH(OH)-CH_2-$ ist, eine Verbindung der Formel I oder ein Tautomer davon, worin $R^2$ Wasserstoff oder Acyloxy und Y $-CO-CH_2-$ ist, mit einem komplexen Metallhydrid reduziert oder

c) zur Herstellung einer Verbindung der Formel I oder einem Tautomer davon, worin $R^2$ Wasserstoff oder Acyloxy und Y $-CH_2-CH_2-$ ist, die Hydroxygruppe in einer Verbindung der Formel I oder einem Tautomer davon, worin $R^2$ Wasserstoff oder Acyloxy und Y $-CH(OH)-CH_2-$ ist, durch Wasserstoff ersetzt oder

d) zur Herstellung einer Verbindung der Formel I oder einem Tautomer davon, worin Y -SO- oder $-SO_2-$ ist, eine Verbindung der Formel

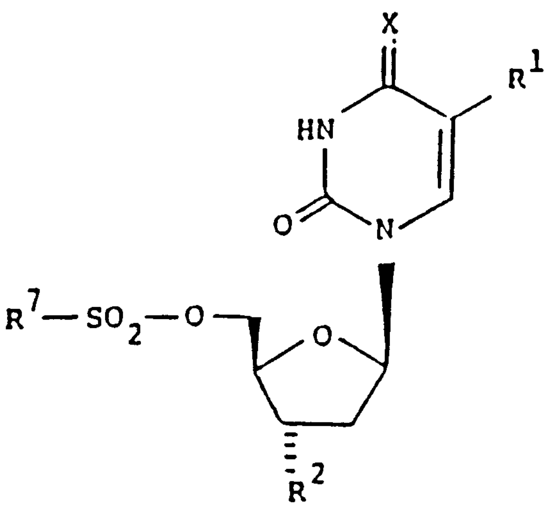

III

worin $R^1$, $R^2$ und Y die obige Bedeutung haben und $R^7$ $C_{1-4}$-Alkyl oder Aryl ist, oder ein Tautomer davon mit einem Alkalimetallderivat einer Verbindung der allgemeinen Formel

$HSC(R^3,R^4,R^5)$ IV

worin $R^3$, $R^4$ und $R^5$ die obige Bedeutung haben, bei erhöhter Temperatur umsetzt und die erhaltene Verbindung der Formel I oder ein erhaltenes Tautomer davon, worin Y -S- ist, oxydiert oder

e) zur Herstellung einer Verbindung der Formel I oder einem Tautomer davon, worin $R^2$ Hydroxy ist, eine Verbindung der Formel I oder ein Tautomer davon, worin $R^2$ Acyloxy ist, deacyliert.

16. Verfahren gemäss Anspruch 15, dadurch gekennzeichnet, dass man gemäss Ausführungsform (a), (b), (c), und/oder (e) verfährt.

17. Pharmazeutische, insbesondere anti-viral wirksame Präparate, enthaltend eine Verbindung gemäss den Ansprüchen 1-12 und ein pharmazeutisches Trägermaterial.

18. Verwendung einer Verbindung gemäss den Ansprüchen 1-12 zur Herstellung von Arzneimitteln zur Kontrolle oder Prophylaxe viraler Infektionen.

**Patentansprüche für folgende Vertragsstaaten : AT, ES, GR**

1. Verfahren zur Herstellung von Verbindungen der allgemeinen Formel

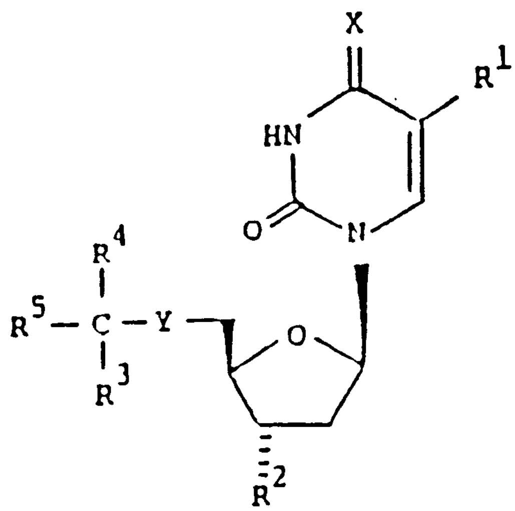

I

worin $R^1$ Halogen, $C_{1-4}$-Alkyl oder Halo-($C_{1-4}$-Alkyl), $R^2$ Wasserstoff, Hydroxy $C_{1-4}$-Alkanoyloxy, Cyclopentylpropionyloxy, Phenacetoxy oder Benzoyloxy, $R^3$ und $R^4$ Wasserstoff oder $C_{1-4}$-Alkyl, $R^5$ Aryl oder Aryloxy, wobei Aryl gegebenenfalls durch einen oder mehrere Substituenten aus der Gruppe Halogen, OH, $C_{1-4}$-Alkyl, $C_{1-4}$-Alkoxy, $CF_3$, $NO_2$ oder Phenyl substituiertes Phenyl bezeichnet, X Sauerstoff oder NH und Y -CO-$CH_2$-, -CH(OH)-$CH_2$-, -$CH_2CH_2$-, -SO- oder -$SO_2$-darstellen,
und Tautomere davon, dadurch gekennzeichnet, dass man

a) zur Herstellung einer Verbindung der Formel I oder einem Tautomer davon, worin $R^2$ Wasserstoff oder Acyloxy und Y -CO-$CH_2$- ist, eine Verbindung der Formel

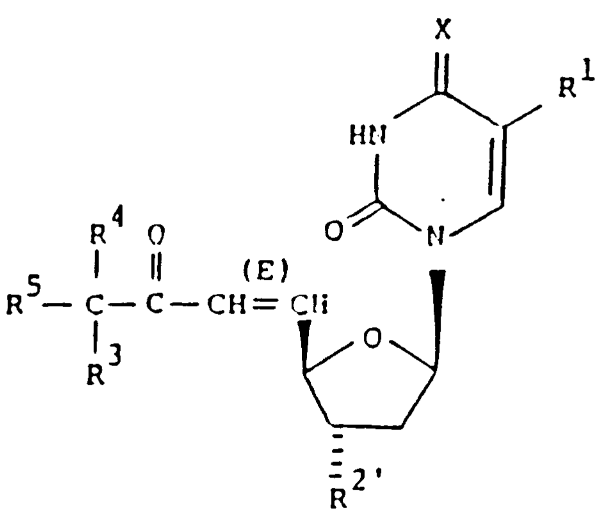

II

worin $R^1$, $R^3$, $R^4$, $R^5$ und X die obige Bedeutung haben und $R^{2'}$ Wasserstoff oder Acyloxy ist,

oder ein Tautomer davon, katalytisch hydriert oder

b) zur Herstellung einer Verbindung der Formel I oder einem Tautomer davon, worin $R^2$ Wasserstoff oder Acyloxy und Y $-CH(OH)-CH_2-$ ist, eine Verbindung der Formel I oder ein Tautomer davon, worin $R^2$ Wasserstoff oder Acyloxy und Y $-CO-CH_2-$ ist, mit einem komplexen Metallhydrid reduziert oder

c) zur Herstellung einer Verbindung der Formel I oder einem Tautomer davon, worin $R^2$ Wasserstoff oder Acyloxy und Y $-CH_2-CH_2-$ ist, die Hydroxygruppe in einer Verbindung der Formel I oder einem Tautomer davon, worin $R^2$ Wasserstoff oder Acyloxy und Y $-CH(OH)-CH_2-$ ist, durch Wasserstoff ersetzt oder

d) zur Herstellung einer Verbindung der Formel I oder einem Tautomer davon, worin Y -SO- oder $-SO_2-$ ist, eine Verbindung der Formel

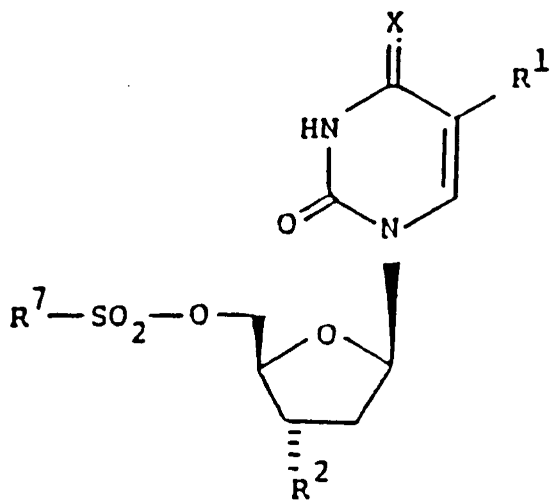

III

worin $R^1$, $R^2$ und Y die obige Bedeutung haben und $R^7$ $C_{1-4}$-Alkyl oder Aryl ist,
oder ein Tautomer davon mit einem Alkalimetallderivat einer Verbindung der allgemeinen Formel

$HSC(R^3,R^4,R^5)$ IV

worin $R^3$, $R^4$ und $R^5$ die obige Bedeutung haben, bei erhöhter Temperatur umsetzt und die erhaltene Verbindung der Formel I oder ein erhaltenes Tautomer davon, worin Y -S- ist, oxydiert oder

e) zur Herstellung einer Verbindung der Formel I oder einem Tautomer davon, worin $R^2$ Hydroxy ist, eine Verbindung der Formel I oder ein Tautomer davon, worin $R^2$ Acyloxy ist, deacyliert.

**2.** Verfahren gemäss Anspruch 1, worin $R^5$ Aryl ist.

**3.** Verfahren gemäss Anspruch 1 oder 2, worin $R^1$ $C_{1-4}$-Alkyl ist.

**4.** Verfahren gemäss den Ansprüchen 1-3, worin $R^2$ Hydroxy oder $C_{1-4}$-Alkanoyloxy ist.

**5.** Verfahren gemäss den Ansprüchen 1-4, worin $R^3$ und $R^4$ Wasserstoff sind.

**6.** Verfahren gemäss den Ansprüchen 1-5, worin $R^5$ Dihalophenyl ist.

**7.** Verfahren gemäss den Ansprüchen 1-6, worin X Sauerstoff ist.

**8.** Verfahren gemäss den Ansprüchen 1-7 worin Y $-COCH_2-$, $-CH(OH)-CH_2-$, $-CH_2-CH_2-$ oder $-SO_2-$ ist.

**9.** Verfahren gemäss den Ansprüchen 1-8, worin $R^1$ Aethyl, $R^2$ Hydroxy oder Acetoxy, $R^3$ und $R^4$ Wasserstoff, $R^5$ 2,6-Dichlorphenyl, X Sauerstoff und Y $-COCH_2-$ oder $-CH(OH)-CH_2-$ ist.

**10.** Verfahren gemäss Anspruch 1, zur Herstellung von

3'-O-Acetyl-5'-[3-(2,6-dichlorphenyl)-2-oxopropyl]-2',5'-dideoxy-5-äthyluridin,
5'-[3-(2,6-Dichlorphenyl)-2-oxopropyl]-2',5'-dideoxy-5-äthyluridin und
5'-[3-(2,6-Dichlorphenyl)-2(RS)-hydroxypropyl]-2',5'-dideoxy-5-äthyluridin.

**11.** Verfahren gemäss Anspruch 2, zur Herstellung von

3'-O-Acetyl-2',5'-dideoxy-5-äthyl-5'-[3-(2,6-dimethylphenyl)-2-oxopropyl]uridin,
3'-O-Acetyl-2',5'-dideoxy-5-äthyl-5'-[3-(2-methylphenyl)-2-oxopropyl]uridin,
3'-O-Acetyl-5'-[3-(2-chlorophenyl)-2-oxopropyl]2',5'-dideoxy-5-äthyluridin,
3'-O-Acetyl-2',5'-dideoxy-5-äthyl-5'-(2-oxo-3-phenylpropyl)uridin,
3'-O-Acetyl-2',5'-dideoxy-5-äthyl-5'-[2-oxo-3(RS)-phenylbutyl]uridin,
2',5'-Dideoxy-5-äthyl-5'-(2-oxo-3-phenylpropyl)uridin,
2',5'-Dideoxy-5-äthyl-5'-[3-(2-methylphenyl)-2-oxopropyl]uridin,
2',5'-Dideoxy-5-äthyl-5'-[3-(2,6-dimethylphenyl)-2-oxopropyl]uridin,
5'-[3-(2-Chlorophenyl)-2-oxopropyl]-2',5'-dideoxy-5-äthyluridin,
2',5'-Dideoxy-5-äthyl-5'-[2-oxo-3(RS)-phenylbutyl]uridin,
2',5'-Dideoxy-5-äthyl-5'-[2(RS)-hydroxy-3-phenylpropyl]uridin,
2',5'-Dideoxy-5-äthyl-5'-(2(RS)-hydroxy-3-(2-methylphenyl)propyl]uridin,
5'-[3-(2-Chlorophenyl)-2(RS)-hydroxypropyl]-2',5'-dideoxy-5-äthyluridin,
2',5'-Dideoxy-5-äthyl-5'-[2(RS)-hydroxy-3-(2,6-dimethylphenyl)propyl]uridin,
2',5'-Dideoxy-5-äthyl-5'-(3-phenylpropyl)uridin und
5'-Benzylsulphonyl-2',5'-dideoxy-5-äthyluridin.

**12.** Verfahren gemäss Anspruch 1, zur Herstellung von

2',5'-Dideoxy-5'-[3-(2,6-dimethylphenyl)-2-oxopropyl]uridin,
3'-O-Acetyl-5'-[3(RS)-(2,4-dichlorphenoxy)-2-oxobutyl]-2',5'-dideoxy-5-äthyluridin,
5'-[3(RS)-(2,4-Dichlorphenoxy)-2-oxobutyl]-2',5'-dideoxy-5-äthyluridin,
5'-[3(RS)-(2,4-Dichlorphenoxy)-2(RS)-hydroxybutyl]-2',5'-dideoxy-5-äthyluridin,
5'-(2-Chlorobenzylsulphonyl)-2',5'-dideoxy-5-äthyluridin,
5'-(2,4-Dichlorbenzylsulphonyl)-2',5'-dideoxy-5-äthyluridin und
5'-(2,6-Dichlorbenzylsulphonyl)-2',5'-dideoxy-5-äthyluridin

**13.** Verfahren gemäss Anspruch 1, dadurch gekennzeichnet, dass man gemäss Ausführungsform (a), (b), (c), und/oder (e) verfährt.

**14.** Verfahren zur Herstellung pharmazeutischer Präparate, insbesondere solcher mit anti-viraler Wirksamkeit, dadurch gekennzeichnet, dass man eine Verbindung der Formel I oder ein Tautomer davon in eine galenische Verabreichungsform bringt.

**15.** Verwendung einer Verbindung der Formel I oder eines Tautomeren davon zur Herstellung von Arzneimitteln zur Kontrolle oder Prophylaxe viraler Infektionen.

**Claims**

**Claims for the following Contracting States : BE, CH, DE, FR, GB, IT, LI, LU, NL, SE**

**1.** Compounds of the general formula

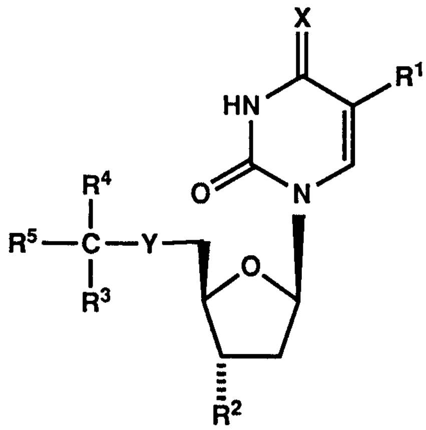

I

wherein $R^1$ represents halogen, $C_{1-4}$-alkyl or halo-($C_{1-4}$-alkyl), $R^2$ represents hydrogen, hydroxy, $C_{1-4}$-alkanoyloxy, cyclopentylpropionyloxy, phenacetoxy or benzoyloxy, $R^3$ and $R^4$ represent hydrogen or $C_{1-4}$-alkyl, $R^5$ represents aryl or aryloxy, whereby aryl denotes phenyl optionally substituted by one or more substituents from the group halogen, OH, $C_{1-4}$-alkyl, $C_{1-4}$-alkoxy, $CF_3$, $NO_2$ or phenyl, X represents O or NH and Y represents $-CO-CH_2-$, $-CH(OH)-CH_2-$, $-CH_2-CH_2-$, -SO- or $-SO_2-$,
and tautomers thereof.

**2.** Compounds according to claim 1, wherein $R^5$ is aryl.

**3.** Compounds according to claim 1 or 2, wherein $R^1$ is $C_{1-4}$-alkyl.

**4.** Compounds according to claims 1-3, wherein $R^2$ is hydroxy or $C_{1-4}$-alkanoyloxy.

**5.** Compounds according to claims 1-4, wherein $R^3$ and $R^4$ are hydrogen.

**6.** Compounds according to claims 1-5, wherein $R^5$ is dihalophenyl.

**7.** Compounds according to claims 1-6, wherein X is oxygen.

**8.** Compounds according to claims 1-7, wherein Y is $-COCH_2-$, $-CH(OH)-CH_2-$, $-CH_2-CH_2-$ or $-SO_2-$.

**9.** Compounds according to claims 1-8, wherein $R^1$ is ethyl, $R^2$ is hydroxy or acetoxy, $R^3$ and $R^4$ are hydrogen, $R^5$ is 2,6-dichlorophenyl, X is oxygen and Y is $-COCH_2-$ or $-CH(OH)-CH_2-$.

**10.** The compounds according to claim 1,

3'-O-acetyl-5'-[3-(2,6-dichlorophenyl)-2-oxopropyl]-2',5'-dideoxy-5-ethyluridine,
5'-[3-(2,6-dichlorophenyl)-2-oxopropyl]-2',5'-dideoxy-5-ethyluridine and
5'-[3-(2,6-dichlorophenyl)-2(RS)-hydroxypropyl]-2',5'-dideoxy-5-ethyluridine.

**11.** The compounds according to claim 2,

3'-O-acetyl-2',5'-dideoxy-5-ethyl-5'-[3-(2,6-dimethylphenyl)-2-oxopropyl]uridine,
3'-O-acetyl-2',5'-dideoxy-5-ethyl-5'-[3-(2-methylphenyl)-2-oxopropyl]uridine,
3'-O-acetyl-5'-[3-(2-chlorophenyl)-2-oxopropyl]-2',5'-dideoxy-5-ethyluridine.
3'-O-acetyl-2',5'-dideoxy-5-ethyl-5'-(2-oxo-3-phenylpropyl)uridine,
3'-O-acetyl-2',5'-dideoxy-5-ethyl-5'-[2-oxo-3(RS)phenylbutyl]uridine,
2',5'-dideoxy-5-ethyl-5'-(2-oxo-3-phenylpropyl)uridine,
2',5'-dideoxy-5-ethyl-5'-[3-(2-methylphenyl)-2-oxopropyl]uridine,
2',5'-dideoxy-5-ethyl-5'-[3-(2,6-dimethylphenyl)-2-oxopropyl]uridine,
5'-[3-(2-chlorophenyl)-2-oxopropyl]-2',5'-dideoxy-5-ethyluridine,
2',5'-dideoxy-5-ethyl-5'-[2-oxo-3(RS)-phenylbutyl]uridine,

2',5'-dideoxy-5-ethyl-5'-[2(RS)-hydroxy-3-phenylpropyl]uridine,
2',5'-dideoxy-5-ethyl-5'-[2(RS)-hydroxy-3-(2-methylphenyl)propyl]uridine,
5'-[3-(2-chlorophenyl)-2(RS)-hydroxypropyl]-2',5'-dideoxy-5-ethyluridine,
2',5'-dideoxy-5-ethyl-5'-[2(RS)-hydroxy-3-(2,6-dimethylphenyl)propyl]uridine,
2',5'-dideoxy-5-ethyl-5'-(3-phenylpropyl)uridine and
5'-benzylsulphonyl-2',5'-dideoxy-5-ethyluridine.

**12.** The compounds according to claim 2,

2',5'-dideoxy-5'-[3-(2,6-dimethylphenyl)-2-oxopropyl]uridine,
3'-O-acetyl-5'-[3(RS)-(2,4-dichlorophenoxy)-2-oxobutyl]-2',5'-dideoxy-5-ethyluridine,
5'-[3(RS)-(2,4-dichlorophenoxy)-2-oxobutyl]-2',5'-dideoxy-5-ethyluridine,
5'-[3(RS)-(2,4-dichlorophenoxy)-2(RS)-hydroxybutyl]-2',5'-dideoxy-5-ethyluridine,
5'-(2-chlorobenzylsulphonyl)-2',5'-dideoxy-5-ethyluridine,
5'-(2,4-dichlorobenzylsulphonyl)-2',5'-dideoxy-5-ethyluridine and
5'-(2,6-dichlorobenzylsulphonyl)-2',5'-dideoxy-5-ethyluridine

**13.** Compounds of the general formula

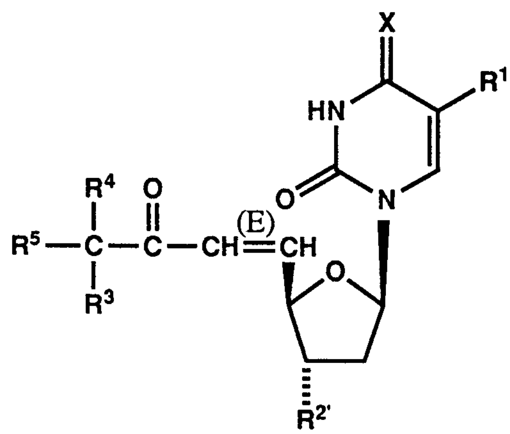

II

wherein $R^1$ is halogen, $C_{1-4}$-alkyl or halo-($C_{1-4}$-alkyl); $R^{2'}$ is hydrogen or acyloxy group; $R^3$ and $R^4$ are hydrogen or $C_{1-4}$-alkyl; $R^5$ is aryl or aryloxy; and X is oxygen or NH, and tautomer thereof.

**14.** The compounds according to claims 1-12 for use as medicaments, especially as medicaments against viruses.

**15.** A process for the manufacture of the compounds of formula I and their tautomer, characterized by
a) for the manufacture of a compound of formula I or a tautomer thereof in which $R^2$ is hydrogen or acyloxy and Y is -CO-$CH_2$-, catalytically hydrogenating a compound of the formula

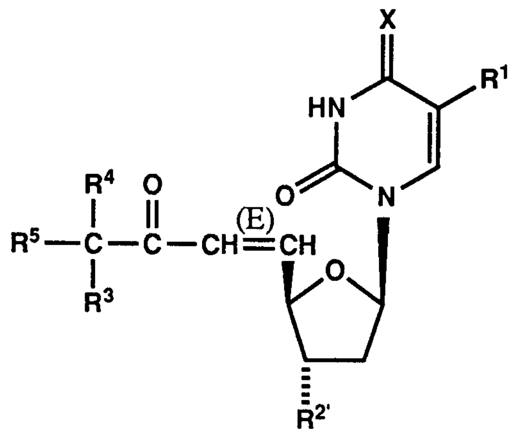

II

wherein $R^1$, $R^3$, $R^4$, $R^5$ and X have the above significance and $R^{2'}$ is hydrogen or acyloxy, or a tautomer thereof, or

b) for the manufacture of a compound of formula I or a tautomer thereof in which $R^2$ is hydrogen or acyloxy and Y is $-CH(OH)-CH_2-$, reducing a compound of formula I or a tautomer thereof in which $R^2$ is hydrogen or acyloxy and Y is $-CO-CH_2-$ with a complex metal hydride, or

c) for the manufacture of a compound of formula I or a tautomer thereof in which $R^2$ is hydrogen or acyloxy and Y is $-CH_2-CH_2-$, replacing the hydroxy group in a compound of formula I or a tautomer thereof in which $R^2$ is hydrogen or acyloxy and Y is $-CH(OH)-CH_2-$ by hydrogen, or

d) for the manufacture of a compound of formula I or a tautomer thereof in which Y is -SO- or $-SO_2-$, reacting a compound of the formula

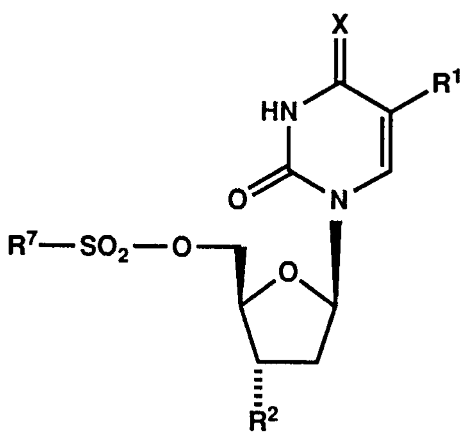

III

wherein $R^1$, $R^2$ and Y have the above significance and $R^7$ is $C_{1-4}$-alkyl or aryl, or a tautomer thereof, with an alkali metal derivative of a compound of the general formula

$HSC(R^3,R^4,R^5)$ IV

wherein $R^3$, $R^4$ and $R^5$ have the above significance, at an elevated temperature and oxidizing the resulting compound of formula I or a tautomer thereof obtained in which Y is -S-, or

e) for the manufacture of a compound of formula I or a tautomer thereof in which $R^2$ is hydroxy, deacylating a compound of formula I or a tautomer thereof in which $R^2$ is acyloxy.

**16.** A process according to claim 15, characterized in that process embodiment (a), (b), (c) and/or (e) is used.

**17.** Pharmaceutical preparations, especially with anti-viral activity, containing a compound according to

claims 1-12 and a pharmaceutical carrier.

**18.** The use of a compound according to claims 1-12 for the manufacture of medicaments for the control or prophylaxis of viral infections.

**Claims for the following Contracting States : AT, ES, GR**

**1.** A process for the manufacture of compounds of the general formula

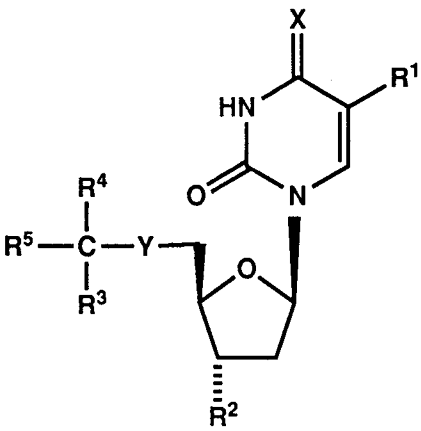

I

wherein $R^1$ represents halogen, $C_{1-4}$-alkyl or halo-($C_{1-4}$-alkyl), $R^2$ represents hydrogen, hydroxy, $C_{1-4}$-alkanoyloxy, cyclopentylpropionyloxy, phenacetoxy or benzoyloxy, $R^3$ and $R^4$ represent hydrogen or $C_{1-4}$-alkyl, $R^5$ represents aryl or aroyloxy, whereby aryl denotes phenyl optionally substituted by one or more substituents from the group halogen, OH, $C_{1-4}$-alkyl, $C_{1-4}$-alkoxy, $CF_3$, $NO_2$ or phenyl, X represents oxygen or NH and Y represents $-CO-CH_2-$, $-CH(OH)-CH_2-$, $-CH_2-CH_2-$, -SO- or $-SO_2-$, and tautomers thereof, characterized by

a) for the manufacture of a compound of formula I or a tautomer thereof in which $R^2$ is hydrogen or acyloxy and Y is $-CO-CH_2-$, catalytically hydrogenating a compound of the general formula

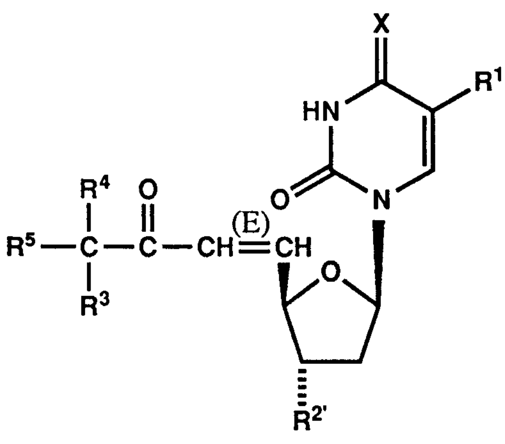

II

wherein $R^1$, $R^3$, $R^4$, $R^5$ and X have the above significance and $R^{2'}$ is hydrogen or acyloxy, or a tautomer thereof, or

b) for the manufacture of a compound of formula I or a tautomer thereof in which $R^2$ is hydrogen or acyloxy and Y is $-CH(OH)-CH_2-$, reducing a compound of formula I or a tautomer thereof in which $R^2$ is hydrogen or acyloxy and Y is $-CO-CH_2-$ with a complex metal hydride, or

c) for the manufacture of a compound of formula I or a tautomer thereof in which $R^2$ is hydrogen or acyloxy and Y is $-CH_2-CH_2-$, replacing the hydroxy group in a compound of formula I or a tautomer thereof in which $R^2$ is hydrogen or acyloxy and Y is $-CH(OH)-CH_2-$ by hydrogen, or

d) for the manufacture of a compound of formula I or a tautomer thereof in which Y is -SO- or $-SO_2-$, reacting a compound of the formula

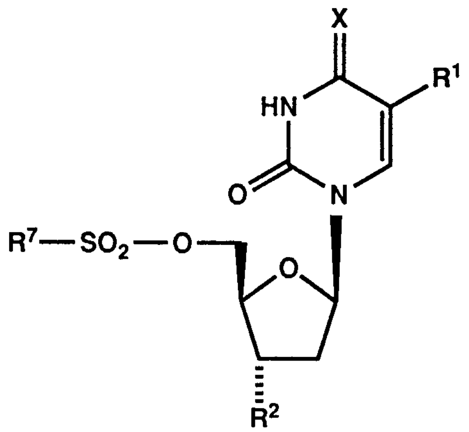

III

wherein $R^1$, $R^2$ and Y have the above significance and $R^7$ is $C_{1-4}$-alkyl or aryl, or a tautomer thereof, with an alkali metal derivative of a compound of the general formula

$HSC(R^3,R^4,R^5)$ IV

wherein $R^3$, $R^4$ and $R^5$ have the above significance, at an elevated temperature and oxidizing the resulting compound of formula I or a tautomer thereof obtained in which Y is -S-, or
e) for the manufacture of a compound of formula I or a tautomer thereof in which $R^2$ is hydroxy, deacylating a compound of formula I or a tautomer thereof in which $R^2$ is acyloxy.

**2.** A process according to claim 1, wherein $R^5$ is aryl.

**3.** A process according to claim 1 or 2, wherein $R^1$ is $C_{1-4}$-alkyl.

**4.** A process according to claim 1-3, wherein $R^2$ is hydroxy or $C_{1-4}$-alkanoyloxy.

**5.** A process according to claims 1-4, wherein $R^3$ and $R^4$ are hydrogen.

**6.** A process according to claims 1-5, wherein $R^5$ is dihalophenyl.

**7.** A process according to claims 1-6, wherein X is oxygen.

**8.** A process according to claims 1-7, wherein Y is $-COCH_2-$, $-CH(OH)-CH_2-$, $-CH_2-CH_2-$ or $-SO_2-$,

**9.** A process according to claims 1-8, wherein $R^1$ is ethyl, $R^2$ is hydroxy or acetoxy, $R^3$ and $R^4$ are hydrogen, $R^5$ is 2,6-dichlorophenyl, X is oxygen and Y is $-COCH_2-$ or $-CH(OH)-CH_2-$.

**10.** A process according to claim 1 for the manufacture of

3'-O-acetyl-5'-[3-(2,6-dichlorophenyl)-2-oxopropyl]-2',5'-dideoxy-5-ethyluridine,
5'-[3-(2,6-dichlorophenyl)-2-oxopropyl]-2',5'-dideoxy-5-ethyluridine and
5'-[3-(2,6-dichlorophenyl)-2(RS)-hydroxypropyl]-2',5'-dideoxy-5-ethyluridine.

**11.** A process according to claim 2 for the manufacture of

3'-O-acetyl-2',5'-dideoxy-5-ethyl-5'-[3-(2,6-dimethylphenyl)-2-oxopropyl]uridine,
3'-O-acetyl-2',5'-dideoxy-5-ethyl-5'-[3-(2-methylphenyl)-2-oxopropyl]uridine,
3'-O-acetyl-5'-[3-(2-chlorophenyl)-2-oxopropyl]-2',5'-dideoxy-5-ethyluridine,
3'-O-acetyl-2',5'-dideoxy-5-ethyl-5'-(2-oxo-3-phenylpropyl)uridine,
3'-O-acetyl-2',5'-dideoxy-5-ethyl-5'-[2-oxo-3(RS)-phenylbutyl]uridine,

2',5'-dideoxy-5-ethyl-5'-(2-oxo-3-phenylpropyl)uridine,
2',5'-dideoxy-5-ethyl-5'-[3-(2-methylphenyl)-2-oxopropyl]uridine,
2',5'-dideoxy-5-ethyl-5'-[3-(2,6-dimethylphenyl)-2-oxopropyl]uridine,
5'-(3-(2-chlorophenyl)-2-oxopropyl]-2',5'-dideoxy-5-ethyluridine,
2',5'-dideoxy-5-ethyl-5'-[2-oxo-3(RS)-phenylbutyl]uridine,
2',5'-dideoxy-5-ethyl-5'-[2(RS)-hydroxy-3-phenylpropyl]uridine,
2',5'-dideoxy-5-ethyl-5'-[2(RS)-hydroxy-3-(2-methylphenyl)propyl]uridine,
5'-[3-(2-chlorophenyl)-2(RS)-hydroxypropyl]-2',5'-dideoxy-5-ethyluridine,
2',5'-dideoxy-5-ethyl-5'-[2(RS)-hydroxy-3-(2,6-dimethylphenyl)propyl]uridine,
2',5'-dideoxy-5-ethyl-5'-(3-phenylpropyl)uridine and
5'-benzylsulphonyl-2',5'-dideoxy-5-ethyluridine.

**12.** A process according to claim 1 for the manufacture of

2',5'-dideoxy-5'-[3-(2,6-dimethylphenyl)-2-oxopropyl]uridine,
3'-O-acetyl-5'-[3(RS)-(2,4-dichlorophenoxy)-2-oxobutyl]-2',5'-dideoxy-5-ethyluridine,
5'-[3(RS)-(2,4-dichlorophenoxy)-2-oxobutyl]-2',5'-dideoxy-5-ethyluridine,
5'-[3(RS)-(2,4-dichlorophenoxy)-2(RS)-hydroxybutyl]-2',5'-dideoxy-5-ethyluridine,
5'-(2-chlorobenzylsulphonyl)-2',5'-dideoxy-2-ethyluridine,
5'-(2,4-dichlorobenzylsulphonyl)-2',5'-dideoxy-5-ethyluridine and
5'-(2,6-dichlorobenzylsulphonyl)-2',5'-dideoxy-5-ethyluridine.

**13.** A process according to claim 1, characterized in that process embodiment (a), (b), (c) and/or (e) is used.

**14.** A process for the manufacture of pharmaceutical preparations, especially those having anti-viral activity, characterized by bringing a compound of formula I or a tautomer thereof into a galenical administration form.

**15.** The use of a compound of formula I or a tautomer thereof for the manufacture of medicaments for the control or prophylaxis of viral infections.

**Revendications**
**Revendications pour les Etats contractants suivants : BE, CH, DE, FR, GB, IT, LI, LU, NL, SE**

**1.** Composés de formule générale

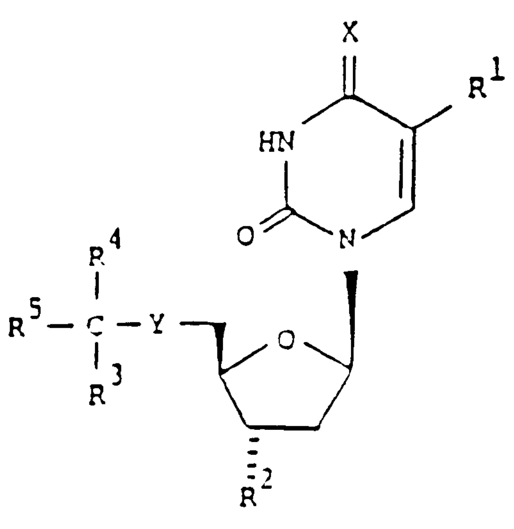

I

où $R^1$ représente un halogène, un alkyle en $C_{1-4}$ ou un halo-(alkyle en $C_{1-4}$), $R^2$ l'hydrogène, un hydroxy, un alcanoyloxy en $C_{1-4}$, le cyclopentylpropionyloxy, le phénacétoxy ou le benzoyloxy, $R^3$ et $R^4$ l'hydrogène ou un alkyle en $C_{1-4}$, $R^5$ un aryle ou un aryloxy, l'aryle désignant un phényle

éventuellement substitué par un ou plusieurs substituants provenant du groupe constitué par un halogène, OH, un alkyle en $C_{1-4}$, un alcoxy en $C_{1-4}$, $CF_3$, $NO_2$ ou le phényle, X l'oxygène ou NH et Y représente $-CO-CH_2-$, $-CH(OH)-CH_2-$, $-CH_2CH_2-$, -SO-ou $-SO_2-$
et leurs tautomères.

**2.** Composés selon la revendication 1 où $R^5$ est l'aryle.

**3.** Composés selon la revendication 1 ou 2 où $R^1$ est un alkyle en $C_{1-4}$.

**4.** Composés selon les revendications 1 à 3 où $R^2$ est l'hydroxy ou un alcanoyloxy en $C_{1-4}$.

**5.** Composés selon les revendications 1 à 4 où $R^3$ et $R^4$ sont l'hydrogène.

**6.** Composés selon les revendications 1 à 5 où $R^5$ est un dihalophényle.

**7.** Composés selon les revendications 1 à 6 où X est l'oxygène.

**8.** Composés selon les revendications 1 à 7 où Y est $-COCH_2-$, $-CH(OH)-CH_2-$, $-CH_2-CH_2-$ ou $-SO_2-$.

**9.** Composés selon les revendications 1 à 8 où $R^1$ est l'éthyle, $R^2$ l'hydroxy ou l'acétoxy, $R^3$ et $R^4$ l'hydrogène, $R^5$ le 2,6-dichlorophényle, X l'oxygène et Y $-COCH_2-$ ou $-CH(OH)-CH_2-$.

**10.** Les composés selon la revendication 1
la 3'-O-acéty1-5'-[3-(2,6-dichlorophény)-2-oxopropyl]-2',5'-didésoxy-5-éthyluridine,
la 5'-[3-(2,6-dichlorophényl)-2-oxopropy]-2',5'-didésoxy-5-éthyluridine et
la 5'-[3-(2,6-dichlorophényl)-2(RS)-hydroxypropyl]-2',5'-didésoxy-5-éthyluridine.

**11.** Les composés selon la revendication 2
la 3'-O-acétyl-2',5'didésoxy-5-éthyl-5'-[3-(2,6-diméthylphényl)-2-oxopropyl]uridine,
la 3'-O-acétyl-2',5'-didésoxy-5-éthyl-5'-[3-(2-méthylphényl)-2-oxopropyl]uridine,
la 3'-O-acétyl-5'-[3-(2-chlorophényl)-2-oxopropyl]-2',5'-didésoxy-5-éthyluridine,
la 3'-O-acétyl-2',5'-didésoxy-5-éthyl-5'-(2-oxo-3-phénylpropyl)uridine,
la 3'-O-acétyl-2',5'-didésoxy-5-éthyl-5'-[2-oxo-3(RS)-phénylbutyl]uridine,
la 2',5'-didésoxy-5-éthyl-5'-(2-oxo-3-phénylpropyl)uridine,
la 2',5'-didésoxy-5-éthyl-5'-[3-(2-méthylphényl)-2-oxopropyl]uridine,
la 2',5'-didésoxy-5-éthyl-5'-[3-(2,6-diméthylphényl)-2-oxopropyl]uridine,
la 5'-[3-(2-chlorophényl)-2-oxopropyl]-2',5'-didésoxy-5-éthyluridine,
la 2',5'-didésoxy-5-éthyl-5'-[2-oxo-3(RS)-phénylbutyl]-uridine,
la 2',5'-didésoxy-5-éthyl-5'-[2(RS)-hydroxy-3-phénylpropyl]-uridine,
la 2',5'-didésoxy-5-éthyl-5'-[2(RS)-hydroxy-3-(2-méthylphényl)propyl]uridine,
la 5'-[3-(2-chlorophényl)-2(RS)-hydroxypropyl]-2',5'-didésoxy-5-éthyluridine,
la 2',5'-didésoxy-5-éthyl-5'-[2(RS)-hydroxy-3-(2,6-diméthylphényl)propyl]uridine,
la 2',5'-didésoxy-5-éthyl-5'-(3-phénylpropyl)uridine et
la 5'-benzylsulfonyl-2',5'-didésoxy-5-éthyluridine.

**12.** Les composés selon la revendication 2
la 2',5'-didésoxy-5'-[3-(2,6-diméthylphényl)-2-oxopropyl]uridine,
la 3'-O-acétyl-5'-[3(RS)-(2,4-dichlorophénoxy)-2-oxobutyl]-2',5'-didésoxy-5-éthyluridine,
la 5'-[3(RS)-(2,4-dichlorophénoxy)-2-oxobutyl]-2',5'-didésoxy-5-éthyluridine,
la 5'-[3(RS)-(2,4-dichlorophénoxy)-2(RS)-hydroxybutyl]-2',5'-didésoxy-5-éthyluridine,
la 5'-(2-chlorobenzylsulfonyl)-2',5'-didésoxy-5-éthyluridine,
la 5'-(2,4-dichlorobenzylsulfonyl)-2',5'-didésoxy-5-éthyluridine et
la 5'-(2,6-dichlorobenzylsulfonyl)-2',5'-didésoxy-5-éthyluridine.

**13.** Composés de formule générale

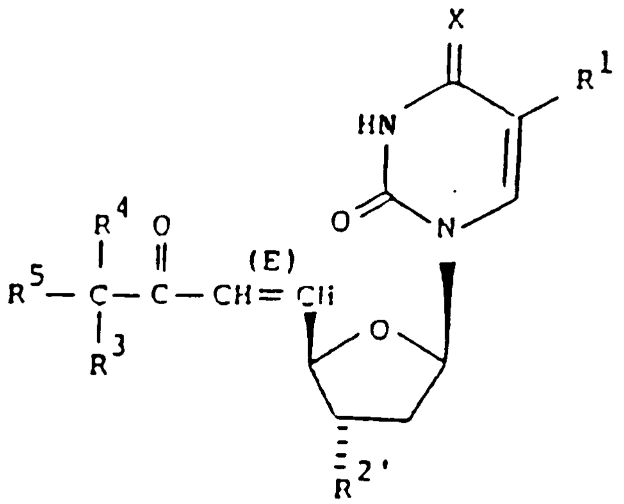

II

où $R^1$ est un halogène, un alkyle en $C_{1-4}$ ou un halo-(alkyle en $C_{1-4}$); $R^2$ est l'hydrogène ou un acyloxy; $R^3$ et $R^4$ sont l'hydrogène ou un alkyle en $C_{1-4}$; $R^5$ est un aryle ou un aryloxy; et X est l'oxygène ou NH
et leurs tautomères.

**14.** Les composés selon les revendications 1 à 12 destinés à être utilisés comme médicaments, en particulier comme médicaments contre les virus.

**15.** Procédé pour la préparation des composés de formule I et de leurs tautomères, caractérisé
a) en ce que, pour la préparation d'un composé de formule I ou de l'un de ses tautomères où $R^2$ est l'hydrogène ou l'acyloxy et Y est $-CO-CH_2-$, on hydrogène catalytiquement un composé de formule

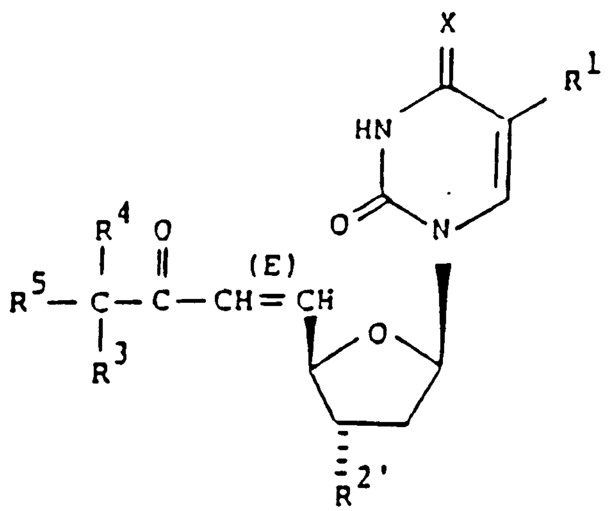

II

où $R^1$, $R^3$, $R^4$, $R^5$ et X ont la signification donnée ci-dessus et $R^2$ est l'hydrogène ou l'acyloxy ou l'un de ses tautomères ou
b) en ce que, pour leur préparation d'un composé de formule I ou de l'un de ses tautomères où $R^2$ est l'hydrogène ou un acyloxy et Y est $-CH(OH)-CH_2-$, on réduit un composé de formule I ou l'un de ses tautomères où $R^2$ est l'hydrogène ou un acyloxy et Y est $-CO-CH_2-$, par un hydrure métallique complexe ou
c) en ce que, pour la préparation d'un composé de formule I ou de l'un de ses tautomères où $R^2$ est l'hydrogène ou un acyloxy et Y est $-CH_2-CH_2-$, on remplace le groupe hydroxy dans un composé de formule I ou dans l'un de ses tautomères où $R^2$ est l'hydrogène ou un acyloxy et Y est $-CH(OH)-CH_2-$, par l'hydrogène ou

d) en ce que, pour la préparation d'un composé de formule I ou de l'un de ses tautomères où Y est -SO- ou -$SO_2$-, on fait réagir, à une température augmentée, un composé de formule

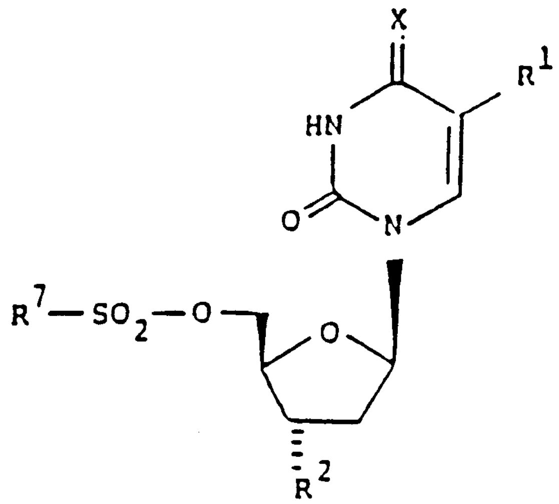

III

où $R^1$, $R^2$ et Y ont la signification donnée ci-dessus et $R^7$ est un alkyle en $C_{1-4}$ ou un aryle, ou de l'un de ses tautomères avec un dérivé de métal alcalin d'un composé de formule générale

HSC($R^3$, $R^4$, $R^5$) IV

ou $R^3$, $R^4$ et $R^5$ ont la signification donnée ci-dessus et en ce que l'on oxyde le composé de formule I obtenu ou l'un de ses tautomères obtenu où Y est -S- ou

e) en ce que, pour la préparation d'un composé de formule I ou de l'un de ses tautomères où $R^2$ est l'hydroxy, on désacyle un composé de formule I ou l'un de ses tautomères où $R^2$ est un acyloxy.

**16.** Procédé selon la revendication 15, caractérisé en ce que l'on procède selon les modes de réalisation (a), (b), (c) et/ou (e).

**17.** Préparations pharmaceutiques, en particulier préparations pharmaceutiques présentant une activité antivirale, contenant un composé selon les revendications 1 à 12 et un support pharmaceutique.

**18.** Utilisation d'un composé selon l'une des revendications 1 à 12 pour la préparation de médicaments destinés au contrôle ou à la prophylaxie des infections virales.

**Revendications pour les Etats contractants suivants : AT, ES, GR**

**1.** Procédé pour la préparation des composés de formule générale

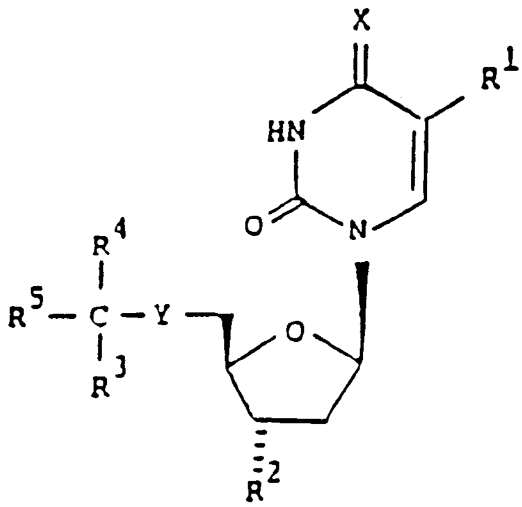

I

où $R^1$ représente un halogène, un alkyle en $C_{1-4}$ ou un halo-(alkyle en $C_{1-4}$), $R^2$ l'hydrogène, un hydroxy, un alcanoyloxy en $C_{1-4}$, le cyclopentylpropionyloxy, le phénacétoxy ou le benzoyloxy, $R^3$ et $R^4$ l'hydrogène ou un alkyle en $C_{1-4}$, $R^5$ un aryle ou un aryloxy, l'aryle désignant un phényle éventuellement substitué par un ou plusieurs substituants provenant du groupe constitué par un halogène, OH, un alkyle en $C_{1-4}$, un alcoxy en $C_{1-4}$, $CF_3$, $NO_2$ ou le phényle, X l'oxygène ou NH et Y représente $-CO-CH_2-$, $-CH(OH)-CH_2-$, $-CH_2CH_2-$, -SO- ou $-SO_2-$
et de leurs tautomères, caractérisé

a) en ce que, pour la préparation d'un composé de formule I ou de l'un de ses tautomères où $R^2$ est l'hydrogène ou l'acyloxy et Y est $-CO-CH_2-$, on hydrogène catalytiquement un composé de formule

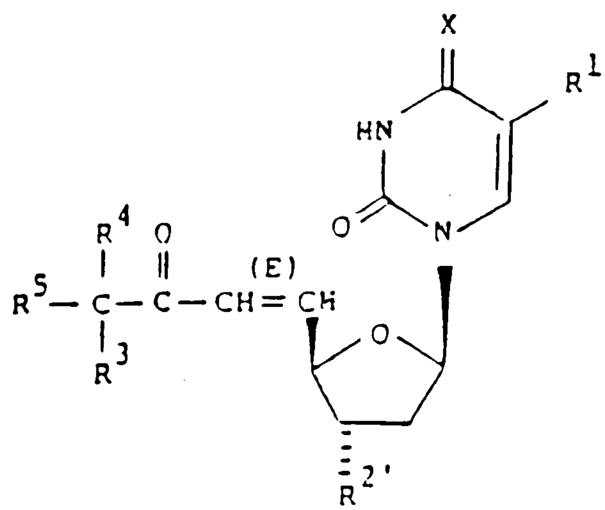

II

où $R^1$, $R^3$, $R^4$, $R^5$ et X ont la signification donnée ci-dessus et $R^{2'}$ est l'hydrogène ou l'acyloxy ou l'un de ses tautomères ou

b) en ce que, pour la préparation d'un composé de formule I ou de l'un de ses tautomères où $R^2$ est l'hydrogène ou un acyloxy et Y est $-CH(OH)-CH_2-$, on réduit un composé de formule I ou l'un de ses tautomères où $R^2$ est l'hydrogène ou un acyloxy et Y est $-CO-CH_2-$, par un hydrure métallique complexe ou

c) en ce que, pour la préparation d'un composé de formule I ou de l'un de ses tautomères où $R^2$ est l'hydrogène ou un acyloxy et Y est $-CH_2-CH_2-$, on remplace le groupe hydroxy dans un composé de formule I ou dans l'un de ses tautomères où $R^2$ est l'hydrogène ou un acyloxy et Y est $-CH(OH)-CH_2-$, par l'hydrogène ou

d) en ce que, pour la préparation d'un composé de formule I ou de l'un de ses tautomères où Y est -SO- ou $-SO_2-$, on fait réagir, à une température augmentée, un composé de formule

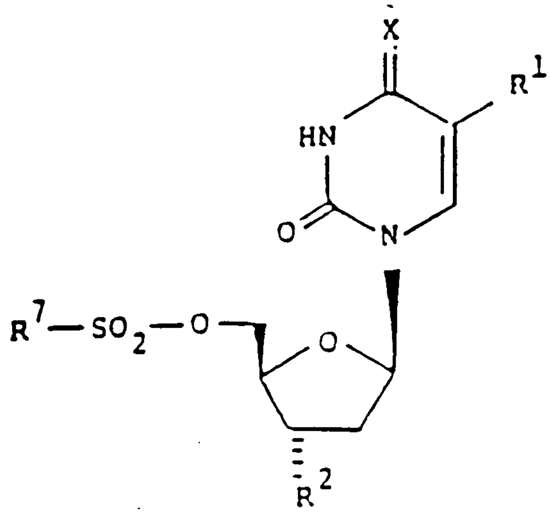

III

où $R^1$ $R^2$ et Y ont la signification donnée ci-dessus et $R^7$ est un alkyle en $C_{1-4}$ ou un aryle, ou de l'un de ses tautomères avec un dérivé de métal alcalin d'un composé de formule générale

HSC($R^3$, $R^4$, $R^5$)    IV

où $R^3$, $R^4$ et $R^5$ ont la signification donnée ci-dessus et en ce que l'on oxyde le composé de formule I obtenu ou l'un de ses tautomères obtenu où Y est -S- ou

e) en ce que, pour la préparation d'un composé de formule I ou de l'un de ses tautomères où $R^2$ est l'hydroxy, on désacyle un composé de formule I ou l'un de ses tautomères où $R^2$ est un acyloxy.

**2.** Procédé selon la revendication 1 où $R^5$ est un aryle.

**3.** Procédé selon la revendication 1 ou 2 où $R^1$ est un alkyle en $C_{1-4}$.

**4.** Procédé selon les revendications 1 à 3 où $R^2$ est l'hydroxy ou un alcanoyloxy en $C_{1-4}$.

**5.** Procédé selon les revendications 1 à 4 où $R^3$ et $R^4$ sont l'hydrogène.

**6.** Procédé selon les revendications 1 à 5 où $R^5$ est un dihalophényle.

**7.** Procédé selon les revendications 1 à 6 où X est l'oxygène.

**8.** Procédé selon les revendications 1 à 7 où Y est $-COCH_2-$, $-CH(OH)-CH_2-$, $-CH_2-CH_2-$ ou $-SO_2-$.

**9.** Procédé selon les revendications 1 à 8 où $R^1$ est l'éthyle, $R^2$ l'hydroxy ou l'acétoxy, $R^3$ et $R^4$ l'hydrogène, $R^5$ le 2,6-dichlorophényle, X l'oxygène et Y est $-COCH_2-$ ou $-CH(OH)-CH_2-$.

**10.** Procédé selon la revendication 1 pour la préparation des
3'-O-acétyl-5'-[3-(2,6-dichlorophényl)-2-oxopropyl]-2',5'-didésoxy-5-éthyluridine,
5'-[3-(2,6-dichlorophényl)-2-oxopropyl]-2',5'-didésoxy-5-éthyluridine et
5'-[3-(2,6-dichlorophényl)-2(RS)-hydroxypropyl]-2',5'-didésoxy-5-éthyluridine.

**11.** Procédé selon la revendication 2 pour la préparation des
3'-O-a c étyl-2',5'-didésoxy-5-éthyl-5'[3-(2,6-diméthylphényl)-2-oxopropyl]uridine,
3'-O-acétyl-2',5'-didésoxy-5-éthyl-5'-[3-(2-méthylphényl)-2-oxopropyl]uridine,
3'-O-acétyl-5'-[3-(2-chlorophényl)-2-oxopropyl]-2',5'-didésoxy-5-éthyluridine,
3'-O-acétyl-2',5'-didésoxy-5-éthyl-5'-(2-oxo-3-phénylpropyl)uridine,
3'-O-acétyl-2',5'-didésoxy-5-éthyl-5'-[2-oxo-3(RS)-phénylbutyl]uridine,
2',5'-didésoxy-5-éthyl-5'-(2-oxo-3-phénylpropyl)uridine,
2',5'-didésoxy-5-éthyl-5'-[3-(2-méthylphényl)-2-oxopropyl]uridine,

2',5'-didésoxy-5-éthyl-5'-[3-(2,6-diméthylphényl)-2-oxopropyl]uridine,
5'-[3-(2-chlorophényl)-2-oxopropyl]-2',5'-didésoxy-5-éthyluridine,
2',5'-didésoxy-5-éthyl-5'-[2-oxo-3(RS)-phénylbutyl]uridine,
2',5,-didésoxy-5-éthyl-5'-[2(RS)-hydroxy-3-phénylpropyl] uridine,
2',5'-didésoxy-5-éthyl-5'-[2(RS)-hydroxy-3-(2-méthylphényl)propyl]uridine,
5'-[3-(2-chlorophényl)-2(RS)-hydroxypropyl]-2',5'-didésoxy-5-éthyluridine,
2',5'-didésoxy-5-éthyl-5'-[2(RS)-hydroxy-3-(2,6-diméthylphényl)propyl]uridine,
2',5'-didésoxy-5-éthyl-5'-(3-phénylpropyl)uridine et
5'-benzylsulfonyl-2'-5'-didésoxy-5-éthyluridine.

**12.** Procédé selon la revendication 1 pour la préparation des
2',5'-didésoxy-5'-[3-(2,6-diméthylphényl-2-oxopropyl]uridine,
3'-O-acétyl-5'-[3(RS)-(2,4-dichlorophénoxy)-2-oxobutyl]-2',5'-didésoxy-5-éthyluridine,
5'-[3(RS)-(2,4-dichlorophénoxy)-2-oxobutyl]-2',5'-didésoxy-5-éthyluridine,
5'-[3(RS)-(2,4-dichlorophénoxy)-2(RS)-hydroxybutyl]-2',5'-didésoxy-5-éthyluridine,
5'-(2-chlorobenzylsulfonyl)-2',5'-didésoxy-5-éthyluridine,
5'-(2,4-dichlorobenzylsulfonyl)-2',5'-didésoxy-5-éthyluridine et
5'-(2,6-dichlorobenzylsulfonyl)-2',5'-didésoxy-5-éthyluridine.

**13.** Procédé selon la revendication 1, caractérisé en ce que l'on procède selon les modes de réalisation (a), (b), (c) et/ou (e).

**14.** Procédé pour l'obtention de préparations pharmaceutiques, en particulier des préparations pharmaceutiques présentant une activité antivirale, caractérisé en ce que l'on met un composé de formule I ou l'un de ses tautomères sous la forme d'une présentation galénique.

**15.** Utilisation d'un composé de formule I ou de l'un de ses tautomères pour la préparation de médicaments destinés au contrôle ou à la prophylaxie des infections virales.